# EUROPEAN PATENT SPECIFICATION

(11) **EP 1 732 878 B1**
(45) Date of publication and mention of the grant of the patent: **18.06.2014**
(21) Application number: 05729187.4
(22) Date of filing: 25.03.2005
(51) Int. Cl.: C07C 209/68

(54) **METHOD FOR THE SYNTHESIS OF QUATERNARY AMMONIUM COMPOUNDS AND COMPOSITIONS THEREOF**
VERFAHREN ZUR SYNTHESE VON QUATERNÄREN AMMONIUMVERBINDUNGEN UND ZUSAMMENSETZUNGEN DAVON
PROCEDE DE SYNTHESE DE COMPOSES D'AMMONIUM QUATERNAIRE ET COMPOSITIONS ASSOCIEES

(30) Priority: 26.03.2004 US 557106 P
(43) Date of publication of application: 20.12.2006
(73) Proprietor: ALBEMARLE CORPORATION, Baton Rouge, Louisiana 70801 (US)
(72) Inventor: SAUER, Joe, D., Baton Rouge, Louisiana 70816 (US); KNIGHT, Christopher, J., Prairieville, Louisiana 70769 (US); EVERLY, Charles, R., Baton Rouge, Louisiana 70816 (US); CHENG, Chi, Hung, Baton Rouge, Louisiana 70810 (US)
(74) Representative: UEXKÜLL & STOLBERG
(86) International application number: PCT/US2005/010162
(87) International publication number: WO 2005/097729

(56) References cited:
- US-A- 5 438 034
- US-A- 5 599 990
- US-A- 5 705 696
- US-B2- 6 586 632
- BRADY J. E. ET AL: "Counterion specificity as the determinant of surfactant aggregation" THE JOURNAL OF PHYSICAL CHEMISTRY, vol. 90, no. 9, 24 April 1986 (1986-04-24), pages 1853-1859, XP002466605 ISSN: 0022-3654
- DATABASE WPI Week 200479 Derwent Publications Ltd., London, GB; AN 2004-800723 XP002466606 & JP 2004 315375 A (MITSUBISHI GAS CHEM CO INC) 11 November 2004 (2004-11-11)
- BRADY ET AL.: "Sontaneous Vesicles", J. AM. CHEM. SOC., vol. 106, 1984, pages 4279-4280,
- TALMON ET AL.: "Spontaneous Vesicles Formed from Hydroxide Surfactants: Evidence from Eectron Microscopy", SCIENCE, vol. 221, 1983, pages 1047-1048,

## Description

### BACKGROUND OF THE INVENTION

### (1) Field of the Invention:

The present invention relates to a process for producing a quaternary amine, and in particular, to a process for producing a quaternary amine that is complexed with a selected anion.

### (2) Description of Related Art:

Wood is an important building and construction material that is used in a variety of applications, such as housing materials, utility poles, and railroad ties. However, wood used in such applications is often exposed to water and soil leading to degradation and staining by molds, fungi, bacteria and insects. Historically, wood has been treated with wood preservatives that contain chemical biocides (preservatives) in order to retard these destructive and unsightly processes. For example, untreated wood left in contact with the ground or water may last from one to four years, while preservative-treated wood has been known to resist microbial decay and insect attack for more than 40 years. As a result, the preservative-treated wood industry is now a $4 billion per year industry, producing approximately 7 billion board feet of treated wood per year.

The three most widely used wood preservatives in the market today are creosote, inorganic arsenic/chromium compounds (CCA), and pentachlorophenol.

More than 95 percent of the preservative-treated wood used in the United States is currently treated with CCA formulations. However, the Environmental Protection Agency (EPA) has announced a decision to phase out the use of CCA preservatives for residential building products in favor of new alternative wood preservatives. As of January 1, 2004, the EPA will not allow CCA products to be used to treat wood intended for virtually all residential uses of wood, including wood used in play-structures, decks, picnic tables, landscaping timbers, residential fencing, patios and walkways/boardwalks. This decision highlights a growing need in the marketplace for new wood preservatives.

The two most widespread alternative wood preservatives are quaternary ammonium compounds (quats) and copper azoles. Because of the marketplace trend away from metal-containing wood preservatives, there has been increased interest in the quats.

Quats are a very diverse group of compounds that find utility not only in the wood preservative/biocide industry, but also in such industries as hair care products, cleaning products, fabric softeners, pharmaceuticals, surfactants, deodorants, mouthwashes, preservatives, emulsifiers, cosmetics, and ore mining. Quats are loosely defined as a group of compounds in which a nitrogen atom is joined to four organic radicals.

Commercial classes of quat compounds have historically developed in a fairly orderly fashion. The general compound alkyltrimethyl ammonium chloride (ATMAC) is representative of class I type quats. These quats are composed of a nitrogen having substituent groups comprising three methyl groups and one alkyl group. Representative of class II quat compounds is the general compound alkyldimethylbenzyl ammonium chloride (ADBAC) - a nitrogen having two substituent methyl groups and in addition, a substituent alkyl group and a substituent alkyl aryl group. The general compound alkyldimethyl(ethylbenzyl) ammonium chloride (ADEBAC) is representative of class III quat compounds. These compounds comprise a nitrogen having substituent groups of two methyl groups, one alkyl group and another alkylaryl group in which the alkyl is other than methyl. Finally, the general compound dialkyldimethyl ammonium chloride (DADMAC) is representative of class IV quat compounds. The class IV quats typically comprise a nitrogen having two methyl group substituents and two alkyl group substituents. One type of class IV quats have substituent alkyl groups of different chain lengths, and can be termed "double-tail" quats. A special class of double-tail quats includes those in which the alkyl substituent groups are of the same chain length. These quats can be referred to as "twin-tail" quats.

Moving from a lower class to a higher class of quats can provide a user with more efficacious surface actives and/or biologically active species. Often, the higher classes of quats are more durable or resistant to deactivation in actual applications. However, moving from a lower class quat to a higher class quat can also result in higher costs per unit of active material.

Higher classes of surface active quats present special challenges for the user and the formulator. Water solubility of higher class quats can be substantially lower than for lower class counterparts. However, solubility can be modified by alteration of the counter-ion (*i.e*., anion) with which the quat is complexed. In many cases, use of "larger" anions can bring the total surface active structure more into balance (*e.g.,* soft acid:soft base combinations). This generally results in a quat salt that displays improved solubility and stability when in aqueous solution.

Methods for the production of quaternary ammonium compounds are generally known in the art. Basic chemistry texts teach that the end-product of the reaction of ammonia or of an amine with an alkyl halide is a quaternary ammonium salt. See, *e.g.,* Noller, C. R., Textbook of Organic Chemistry, 2nd Ed., p.188 et seq., W. B. Saunders Co., Philadelphia (1961), and March, J., Advanced Organic Chemistry, 3rd Ed., pp.364, 365, John Wiley & Sons, New York (1985). Numerous other publications, for example U.S. Patent Nos. 4,444,790, 4,450,174, 5,308,363, 5,399,762, 5,438,034, 5,523,487, 5,559,155, 5,641,726, 5,700,841, 5,760,088, 5,833,741, 5,855,817, 5,891,921, 5,944,880, 6,080,789, 6,087,303, 6,090,855, 6,172,117, 6,180,672, 6,362,370, 6,464,764, 6,485,790, 6,784,300, 6,784,317, and U.S. Patent Application Publications US 2003/0023108, and US 2004/0162343, report specific methods of producing quats having particular types of substituent groups and particular counter-anions.

U.S. Patent No. 2,295,504 to Shelton, for example describes the formation of cetyltriethylammonium salts by reacting triethylamine with cetyl iodide, and states that corresponding stearyl, myristyl and oleyl triethyl ammonium iodides can be made in a similar manner. Similar reactions using alkyl bromides and alkyl chlorides are also reported. The reaction of a quat bromide with silver sulfate to form the quat sulfate and insoluble silver bromide is described. More complex quats are formed by sequential reactions with different alkyl halides, each adding a particular alkyl group to the nitrogen.

De Benneville, in U.S. Patent No. 2,994,699, reports the formation of ketonic quaternary ammonium compounds and states that while it is advantageous to initially prepare the compounds in the form of halides, different anions can be supplied by either metathesis or ion exchange. Examples include conversion of the halide salt to the hydroxyl salt by reaction of the quat halide with silver oxide, or the like, and the subsequent conversion of the hydroxyl to any desired anion form by acidifying with an acid of the desired anion. The patent states that any of the quat salts described therein can be converted to any other anion form by contacting it with an anion-exchange resin in the desired anion form. No examples of an ion exchange anion transfer are provided.

In U.S. Patent No. 3,190,919, Swanson describes two methods of exchanging anions of quat salts. The first method is described as repeatedly contacting an organic solution of the quat with an aqueous solution containing the desired anion. However, the method is described as being cumbersome to carry out commercially, and to be expensive and somewhat hazardous, due to the large amounts of solvents employed. The use of ion exchange resins is described as the second method for exchanging quat anions. Here, the quat is passed through a column of ion exchange resin in the form of the final desired quat anion. When it is said that an ion exchange resin is in the form of a particular anion, it is meant that the resin is complexed with that anion. The resin releases the desired anion and absorbs the starting anion. Swanson describes the method as being useful on a laboratory scale but highly impractical on a commercial scale, and states that a dilute solution of the quat must be used, requiring large amounts of solvents. In addition, he states that ion exchange resins are expensive and must be periodically replaced. Large quantities of resins must be employed, first because of low capacity inherent in exchange resins and second because of low transfer coefficients attendant with the use of non-protic solvents, such as hydrocarbons. Further, he states, regeneration is very difficult when non-protic solvents are employed. He concludes that the use of an ion exchange resin for exchange of quat anions is highly uneconomical.

To overcome these problems, Swanson describes a process wherein a quat salt having an anion of a volatile acid, such as sulfurous, is dissolved in a non-water soluble organic solvent, such as kerosene, and the organic solution is contacted with an aqueous solution of a non-volatile acid, such as sulfuric. An inert gas - air, for example - is purged through the mixture, stripping out the volatile acid and leaving the quat salt of the non-volatile acid.

In U.S. Patent No. 4,892,944, Mori et al. describe the production of a quat salt by a two-step method involving the reaction of a tertiary amine with a carbonic acid diester to produce a quat carbonate, next, the quat carbonate is mixed with an acid while removing carbon dioxide to product the quat salt of the acid anion.

U.S. Patent No. 5,438,034 to Walker, describes the preparation of quat ammonium carbonate and quat ammonium bicarbonate by reacting a dialkyldimethylammonium chloride and a metal hydroxide in a C₁ - C₄ normal alcohol solvent to form a quat hydroxide. The quat hydroxide is then reacted with carbon dioxide to yield the corresponding quat carbonate and quat bicarbonate. The patent describes the prior art as including the use of an ion exchange resin to convert didecyldimethylammonium bromide to didecyldimethylammonium hydroxide (Talmon et al., Science, 221:1047 (1983)), but states that a large amount of resin was required for conversion of a small amount of quat compound. In fact, the Talmon *et al.* article describes the process as using a hydroxide ion exchange resin in water at 25°C in batch mode to convert a quat bromide, which was insoluble in water, to the corresponding quat hydroxide. The article reported that the quat hyroxide was highly soluble in water and spontaneously formed stable vesicles.

Methods for exchanging anions of quat compounds are described in: U.S. Patent No. 3,523,068 (electrolytic preparation of a quat hydroxide from an aqueous solution of a quat salt of a non-electrolyzable anion, such as sulfate, bisulfate, alkylsulfate, nitrate, carbonate, and bicarbonate); U.S. Patent No. 4,394,226 (electrolytic preparation of quat hydroxide from quat halides in electrolytic cells separated by a cation exchange membrane); U.S. Patent No. 4,634,509 (electrolysis of an inorganic acid quat salt in a diaphragm cell to produce the quat hydroxide); U.S. Patent No. 4,776,929 (production of quat hydroxide by electrolyzing quat bicarbonate salts in a cell having an anode and a cathode compartment separated by a cation exchange membrane); U.S. Patent No. 5,705,696 (producing a quat ammonium salt having a non-halide anion by contacting an aqueous solution of a quat halide and an alkali metal salt of the desired anion with an organic liquid which is immiscible with the aqueous solution and which is a solvent for the desired quat salt); and U.S. Patent No. 6,586,632 (conversion of a quat halide to a quat dihydrogen phosphate or bisulfate salt).

US 5 599 990 discloses preparation of a quaternary ammonium halide by reacting a substituted or unsubstituted haloalkane with a tert.-amine in a mole ratio not higher than 1.1/1 in the absence of a solvent and at a temperature such as to maintain the reaction mass liquid.

Brady et al., "Spontaneous Vesicles", J. Am. Chem. Soc., Vol. 106, 1984, pages 4279-4280, disclose the preparation of didodecyldimethylammonium salts from the corresponding hydroxide salt DDAOH with a stoichiometric amount of the appropriate acid.

As can be seen from the volume of the foregoing work, it would be highly desirable to provide a method to produce a quaternary tetraalkylammonium salt of a selected anion that provides the quat with desirable properties for particular applications. It would be even more useful if such a method could be operated with' a high degree of conversion so that the exchange leaves very low residual content of anions other than the selected anion with the quat compound. It would be yet more useful if such a method could be operated with high efficiency.

BRIEF SUMMARY OF THE INVENTION [00020] Briefly, therefore, the present invention is directed to a novel method of producing a quaternary tetraalkylammonium salt of a second anion, the method comprising:
reacting a trialkylamine with an alkyl bromide to form a quaternary tetraalkylammonium bromide salt;
converting the quaternary tetraalkylammonium bromide salt to a quaternary tetraalkylammonium hydroxide salt by
   - contacting an ion exchange resin in hydroxide form with a solution comprising
      - the quaternary tetraalkylammonium bromide salt in an amount of above at least 10% by weight and
      - a solvent that includes water and a polar organic co-solvent and has a ratio of co-solvent to water of 10:90 to 99:1, and
forming the quaternary, tetraalkylammonium hydroxide salt in solution and the ion exchange resin in bromide form,
whereby contacting the ion exchange resin in hydroxide form with the solution comprising the quaternary tetraalkylammonium bromide salt comprises flowing the solution containing the tetraalkylammonium bromide salt into a vessel containing a bed of the ion exchange resin in hydroxide form so that the solution passes through the resin bed causing the bromide anions to exchange with the hydroxide anions to form quaternary tetraalkylammonium hydroxide salt and ion exchange resin in bromide form; and converting the quaternary tetraalkylammonium hydroxide salt to the quaternary tetraalkylammonium salt of the second anion.

In one embodiment of the present invention, the step of converting the quaternary tetraalkylammonium hydroxide salt to the quaternary tetraalkylammonium salt of the second anion comprises contacting the solution of the tetraatkytammonium hydroxide salt with an ion exchange resin in the form of the second anion and forming a tetraalkylammonium salt of the second anion in solution and the ion exchange resin in hydroxide form.

In another embodiment of the present invention, the step of converting the quaternary tetraalkylammonium hydroxide salt to the quaternary tetraalkylammonium salt of the second anion comprises contacting the tetraalkylammonium hydroxide salt with an acid of the second anion and converting of the tetraalkylammonium hydroxide salt to a tetraalkylammonium salt of the second anion.

The present invention is also directed to a novel method of converting a quaternary tetraalkylammonium hydroxide salt to a quaternary tetraalkylammonium carbonate/bicarbonate salt, the method comprising: feeding a solution comprising a quaternary tetraalkylammonium hydroxide salt in a solvent comprising water and a volatile polar organic co-solvent into a distillation column at an appropriate point; feeding carbon dioxide gas into the column near its bottom; releasing a solution from the bottom of the column which is substantially free of the co-solvent and contains the quaternary tetraalkylammonium carbonate/bicarbonate salt; and releasing a stream from the top of the column comprising the co-solvent.

### BRIEF DESCRIPTION OF THE DRAWINGS

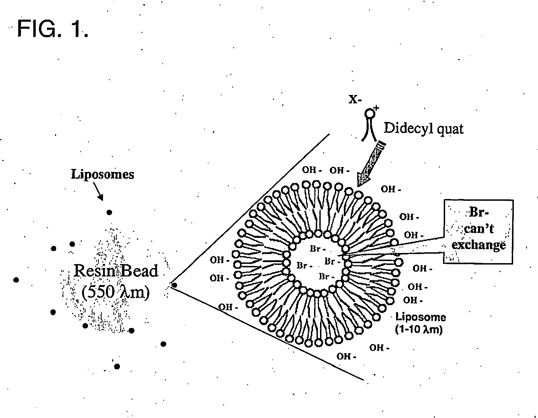

FIG. 1 is an illustration of a molecular assembly, here identified as a liposome, that is composed of a tetraalkylammonium salt of a first anion, which is shown here as didecyldimethylammonium bromide salt, in a liquid solution and in contact with an ion exchange resin bead in hydroxide form, where it is seen that the molecular assembly entraps a certain amount of the bromide ions, making them unavailable for exchange with hydroxide ions;

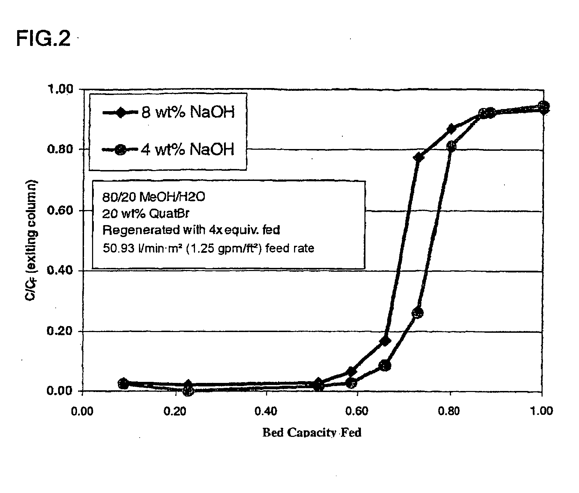

FIG. 2 shows a graph of the weight of bromide present in successive samples of eluant from the mid-point of a bed of Dowex® Marathon A2, Type 2, strong base ion exchange resin (indicating break through of bromide ions past the sample point) as a function of the concentration of sodium hydroxide in aqueous solution used for regeneration; and

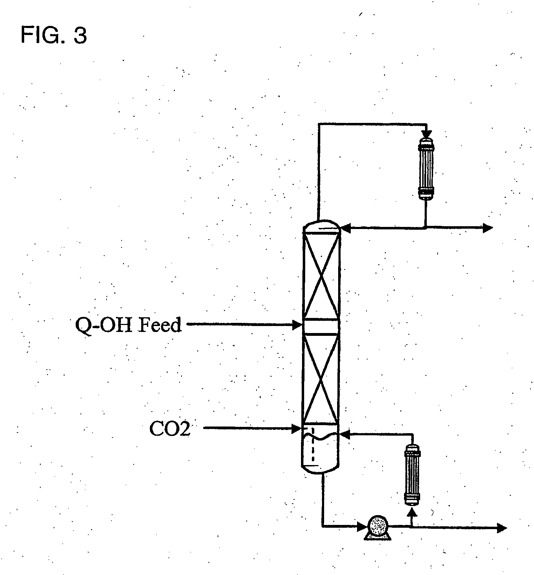

FIG. 3 shows a schematic flow diagram of a reactive distillation column that is useful in an embodiment of the present invention for converting a tetraalkylammonium hydroxide salt to a tetraalkylammonium carbonate and/or bicarbonate salt and for stripping volatile organic solvent from a solution of the quaternary tetraalkylammonium salt.

### DETAILED DESCRIPTION OF THE PREFERRED EMBODIMENTS

In accordance with the present invention, it has been discovered that a quaternary tetraalkylammonium salt of a selected anion (which can be referred to herein as a "second anion") is produced by reacting a trialkylamine with an alkyl bromide to form a quaternary tetraalkylammonium bromide salt. Next, the quaternary tetraalkylammonium bromide salt is converted to a quaternary tetraalkylammonium hydroxide salt by contacting an ion exchange resin in hydroxide form with a solution comprising the quaternary tetraalkylammonium bromide salt and a solvent that includes water and a polar organic co-solvent. This ion exchange contact causes the formation of the quaternary tetraalkylammonium hydroxide salt in solution and the ion exchange resin in bromide form. Finally, the quaternary tetraalkylammonium hydroxide salt is converted to the desired quaternary tetraalkylammonium salt of the selected anion.

One aspect of the present invention allows for the synthesis of quaternary ammonium compounds from alkyldimethylamine (ADMA) compounds such as decyldimethylamine, and avoids the use of more expensive compounds such as dialkylmethylamines (DAMA). This synthesis route provides significant advantages in terms of cost and flexibility. For example, by using an alkyldimethylamine such as decyldimethylamine, the present invention allows for the production of either symmetric or asymmetric quaternary ammonium compounds with regards to the nature and identity of the alkyl groups present in the finished quat without having to resort to a different starting compound for each desired type of quat.

In other aspects, the present invention also has the advantage of providing the ability to exchange the counter-ion (anion) in which form the quat is synthesized, with a selected anion that provides the quat salt with desired properties.

The present method describes the synthesis of quaternary ammonium compounds. As used herein, the term "quaternary ammonium compound" or "quat" refers to a compound in which at least one nitrogen atom is joined to four organic radicals leaving a net positive charge. In certain aspects, the organic radicals can be alkyl or alkenyl (unsaturated alkyls) groups that are linear or branched, substituted or unsubstituted, or mixtures thereof. In other aspects, the term "quaternary ammonium compound" or "quat" is also intended to encompass a compound in which more than one nitrogen is joined to four organic radicals. For example, one of the four organic radicals of a quat may be a "shared" radical with a second quat.

Due to a net positive charge on the nitrogen atom of a quaternary ammonium compound, all quats are normally complexed with an associated counter-ion (*i.e*., an anion). Depending upon the valence charge of the counter-ion, more than one positively-charged quat may be complexed with a particular counter-ion. For example, two positively-charged quats may be complexed with a carbonate counter-ion.

In the present method, a trialkylamine is reacted with an alkyl bromide to form a quaternary tetraalkylammonium bromide salt. Although almost any trialkylamine can be used, it is preferred that the trialkylamine is an alkyldimethylamine.

As used herein, the term "alkyldimethylamine" is intended to encompass any compound having a nitrogen atom joined with two methyl groups and one alkyl group, wherein the alkyl group could be a methyl group or a branched or unbranched or substituted or unsubstituted alkyl or alkenyl group. Although any suitable alkyldimethylamine compound can be used in the present method, in certain embodiments, a C₁-C₂₀ alkyldimethylamine compound is used. In this aspect, the alkyl group of the alkyldimethylamine compound can independently have from one to twenty carbons. Likewise, the alkyl group can be linear or branched, saturated or unsaturated, substituted or unsubstituted, or mixtures thereof. In some embodiments, the alkyldimethylamine is a C₁-C₂₀ alkyldimethylamine; in other embodiments, the alkyldimethylamine is a C₂-C₂₀ alkyldimethylamine; and in still other embodiments, the alkyldimethylamine is a C₈-C₁₂ alkyldimethylamine. In certain aspects of the present invention, the alkyldimethylamine is decyldimethylamine.

As used herein, the term "alkyl bromide" is intended to encompass any bromide compound having an alkyl group or alkenyl group that is the same or different than the alkyl group on the alkyldimethylamine and is joined with one or more bromide groups. Although almost any alkyl bromide compound can be used in the present invention, in certain embodiments, a C₁-C₂₀ alkyl bromide is used. The alkyl group of the alkyl bromide can be linear or branched, saturated or unsaturated, substituted or unsubstituted, or mixtures thereof. In still other embodiments, the alkyl bromide is a C₂-C₂₀ alkyl bromide; in other embodiments, the alkyl bromide is a C₈-C₁₂ alkyl bromide; and in some embodiments, the alkyl bromide is decyl bromide.

In certain aspects of the invention, any one or more of the alkyl group(s) of the alkyldimethylamine and/or the alkyl bromide may be optionally substituted at any carbon thereof with any one or more of a desired substituent group. When present, each of these substituent groups independently comprise at least one or more groups selected from alkyl, aryl, heterocycle, hydroxyl, ester, benzyl, carboxyl, halo, nitro, cyano, alkoxy and oxo.

By way of example, the product of a reaction between decyldimethylamine and decyl bromide is the quaternary ammonium salt - didecyldimethylammonium bromide.

The reaction between the trialkylamine and the alkyl bromide can be performed by adding the alkyl bromide to a solution of the alkyldimethylamine in a reaction vessel that is optionally equipped with a heating mantel and magnetic stirrer. Although the order of reactant addition is not crucial to the operability of the present invention, by way of example, a reaction quantity of an alkyldimethylamine, such as decyldimethylamine, is optionally heated to between 50°C and 65°C or higher under vacuum to purge oxygen from the reaction vessel. The entire reaction may be carried out in an atmosphere of inert gas such as a partial or full nitrogen atmosphere. The reactants may be stirred during the course of the reaction to ensure complete mixing of the reactants.

Once the alkyldimethylamine is added to the reaction vessel and optionally heated to a suitable reaction temperature, the alkyl bromide is added to the reaction vessel. The time for addition of the alkyl bromide is not critical to the operability of the present invention and can be carried out over a time period of up to 5 hours. For example, the addition of the alkyl bromide can be carried out slowly over the course of three hours or, in other embodiments, the entirety of the alkyl bromide can be added to the alkyldimethylamine at one time. In addition, because the reaction between the alkyldimethylamine and the alkyl bromide is an exothermic reaction, it may be suitable to provide the reaction vessel with cooling capabilities to keep the reaction from overheating. For example, without cooling, the reaction can reach temperatures of between 140°C and 180°C. In some instances, the temperature of the reaction is preferably maintained below 120°C. Although any cooling method can be utilized with the present invention, two such methods for controlling the temperature of the reaction are (i) using a cooling jacketed reaction vessel and (ii) adding methanol and then refluxing.

While the quaternary tetralkylammonium bromide salt is forming during the course of the reaction, small quantities of a C₁-C₄ alcohol, and in certain embodiments, methanol, may be added to the reaction vessel to assist in preventing the quaternary salt from precipitating out of the reaction and to control viscosity. Any evaporated methanol may be returned to the reaction mixture via a reflux condenser apparatus.

Optionally, any water present in the reaction may be removed by distillation, by vacuum distillation, or as an ethanol azeotrope prior to the completion of the first step of the reaction. This can be accomplished by adding ethanol to the reaction mixture and removing the water/ethanol azeotrope by distillation.

Generally, about equimolar portions of alkyldimethylamine and alkyl bromide reactants are used, with the overall molar ratio of alkyldimethylamine to alkyl bromide typically being at least 1:1. However, either of the alkyldimethylamine or alkyl bromide may be used in molar excess according to the process of the invention. In certain embodiments, an excess of alkyldimethylamine may be employed so that the molar ratio of alkyldimethylamine to alkyl bromide is between 1.0001:1 to 2:1. A preferred molar ratio of trialkyamine-to-alkyl bromide is from 1.001:1 to 1.2:1 (*i.e*., so that the alkyldimethylamine molar excess is between 0.1% and 20% more than the molar amount of the alkyl bromide), and a molar ratio between 1.01:1 and 1.02:1 is even more preferred (a molar excess of 1% to 2%). In certain embodiments, a slight excess of alkyl bromide may be employed so that the molar ratio of alkyl bromide to alkyldimethylamine is between 1.001:1 to 1.5:1 (*i.e.,* so that the alkyl bromide molar excess is between 0.1% and 50% more than the molar amount of the alkyldimethylamine, preferably between 1% and 30%). For example, in one embodiment, an alkyl bromide molar excess of 1% to 15% can be used.

After the alkyl bromide addition is complete, the reaction is then extended for an additional period of 1 to 24 hours, and in some embodiments the reaction is extended for an additional period of 6 to 12 hours. The reaction is considered complete when less than 2 weight % ("wt. %"), or in some embodiments less than 1 wt. %, of the starting reactants remain. The product formed is a quaternary tetraalkylammonium bromide salt that includes the alkyl group and two methyl groups contributed by the alkyldimethylamine and the fourth alkyl group contributed by the alkyl bromide. An exemplary compound is didecyldimethylammonium bromide.

When the quaternary tetraalkylammonium bromide salt has been formed, it is converted to a quaternary tetraalkylammonium hydroxide salt by contacting an ion exchange resin in hydroxide form with a solution comprising the quaternary tetraalkylammonium bromide salt and a solvent that includes water and a polar organic co-solvent. This ion exchange contact causes the formation of the quaternary tetraalkylammonium hydroxide salt in solution and the ion exchange resin in bromide form.

An advantage of the ion exchange step of the present method is that it is effective at quat concentrations and flow rates that are suitable for commercial industrial applications. For example, quat concentrations of over 10%, or 15%, or even 25% by weight can be processed in the present method. Also, the novel method permits the production of quat hydroxide salts having extremely low residual levels of bromide. For example, product solutions containing less than 1000 ppm, or even less than 300 ppm of bromide, or even lower, are obtainable. Even more useful is the fact that the inventors have found that the ion exchange resin can be regenerated frequently and over many cycles without significant loss in exchange efficiency or capacity.

When the tetraalkylammonium bromide salt is placed into solution, the solution comprises above at least 10 % by weight and up to the solubility limit of the solvent. At least 15% tetraalkylammonium bromide salt by weight is yet more preferred, and at least 20%, or even more preferably at least 25% tetraalkylammonium bromide salt by weight, or an even higher concentration, is even more preferred. In some embodiments of the present invention, it is preferred that the solution contain the quat bromide salt in an amount that is between 10% and 30%, by weight, and between 15% and 25% is even more preferred.

The solution containing the tetraalkylammonium bromide salt can be formed by any method that is effective for such operation. The quat salt can be added to the solvent with stirring until the quat salt goes into solution. If it is helpful, the solvent can be heated somewhat to speed the rate at which the quat salt is solubilized.

The solvent that is used in the present invention to dissolve the tetraalkylammonium bromide salt is one that must meet certain criteria in order for the method to be successful. The quat bromide salts, and quat hydroxide salts are normally amphipathic in nature and exhibit very low true solubility in normal aqueous media. To be practical at a commercial industrial scale of interest, the exchange must be practiced at solute concentrations that are very high when compared with normal solute concentrations in conventional ion exchange operations. In the present case, this means concentrations of the quat salts such as are described below.

in order to be successful, various equilibria that are involved in the present ion exchange process (such as, for example, micellar equilibria, resin-ion to solvent-ion equilibrium, and the like) have to be manipulated such that the ion swap can take place quickly, at high process flow rates, *e.g.,* such as would be required for a successful commercial semi-continuous type of operation. To allow for the mechanics of this overall system to achieve the desired exchanges, the solvent system that is used is of particular importance. The inventors have found that design and selection of the components of the solvent should take into account the following features:
- Water, alone, does not provide for adequate mobility and subsequent exchange of anions. This is believed to be due to the molecular assembly structures established in water by the types of amphipathic solutes involved. Water is a good solvent for the inorganic portion of the quat, but it is not a good solvent for the hydrophobic alkyl tails of some substituent groups;
- Non-aqueous solvents typically are not capable of providing solvent-separated ion pairs that can participate in the desired ion-exchange at reasonable rates. However, organic solvents provide increased solvation of hydrophobictails of higher alkyl substituent groups;

- Double-tailed surfactants, in particular, exhibit low water compatibility and form viscous "gels" at concentrations ranging from parts-per-million levels on up continuously to fairly elevated concentrations (>50%) in water-only environments; and
- Twin-tailed surfactants tend to be special cases within the "double-tail" family. These special examples typically form high viscosity systems in most solvents.

It has been found, however, that solvents can be prepared that meet the various needs of the present method, and permit the desired exchange to proceed quickly and completely. The present solvent is preferably one in which:
(i) the tetraalkylammonium bromide salt is soluble in an amount of above at least 10 % by weight at 25°C,
(ii) the tetraalkylammonium hydroxide salt is soluble in an amount of above at least 10 % by weight at 25°C, and
(iii)the bromide is available for contact with the ion exchange resin. Each of these characteristics of the solvent will now be described in detail.

When it is said that a quat salt is soluble in the solvent, it is meant that the described amount of the quat salt can be dissolved in the solvent when the solvent is at a temperature of 25°C. The solvent is one which can contain above at least 10 % by weight of the tetraalkylammonium bromide salt in solution, and that it is one which can contain above at least 10 % by weight of the tetraalkylammonium hydroxide salt in solution. It is preferred that the solvent is one in which the tetraalkylammonium bromide salt and the tetraalkylammonium hydroxide salt are soluble in an amount of at least at least 15%, by weight is even more preferred, at least 20%, by weight, is yet more preferred, and at least 25%, by weight, or even higher, is even more preferred, where all solubilities are measured at 25°C. It should be understood that when it is said that the solvent should be one in which some amount of the tetraalkylammonium bromide salt is soluble and in which some amount of the tetraalkylammonium hydroxide salt is soluble, it is not meant that those amounts of the two quat salts must be soluble in the same volume of solvent simultaneously, It means that the specified amount of each of the two quat salts must be separately soluble in a specified volume of the solvent.

When it is said that the solvent must be one in which the bromide anion is available for contact with the ion exchange resin, it is meant that the tetraalkylammonium bromide salt does not form molecular assemblies that shield the bromide anion from contact with the ion exchange resin, and also that the tetraalkylammonium bromide salt and the ion exchange resin in hydroxide form are ionized, at least to some degree.

It is known that the tetraalkylammonium bromide salts that are of interest in the present method form molecular assemblies in water that shield the bromide from contact with the ion exchange resin. Such molecular assemblies include, but are not limited to, vesicles, micelles, liposomes, liquid crystal arrays, reverse micelles, and the like. Because critical micelle concentration (CMC) values for the quats salt of use in the present invention in water are very low, on the order of 10⁻³ to 10⁻⁹ molar, or even lower, it is almost certain that such molecular assemblies would occur at the concentrations of quat salts that are of interest in the present method if only water is used as the solvent.

When solvents having high water concentrations are used (low co-colvent : water ratio) it is believed that tetraalkylammonium salts, such as a double-tailed quat, can fold over, shielding some of the first anion from exchange with the resin. This is illustrated in FIG. 1, where a molecular assembly, labeled as a liposome and composed of double-tailed quat salts, didecyldimethylammonium salts in this case, is shown to be shielding an amount of bromide. Unless and until the molecular assembly is disrupted, allowing access of the trapped bromide ions to the resin bead, ion exchange for the shielded bromide cannot occur.

As mentioned above, it is also preferred that the solvent be one in which the quat bromide salt and the ion exchange resin in hydroxide form are ionized, at least to some degree. It is believed that the presence of the anions in solution promotes exchange rate and degree of completeness.

The availability of the bromide anion for contact rivith the ion exchange resin can easily be determined for any particular solvent / quat salt system. One example of a method for this determination is the following test.
1. One mole of a quat bromide salt is dissolved in the solvent to be tested and made up to exactly one liter. The theoretical concentration of the bromide anion is 1 mole/liter.
2. The indicated, or available, concentration of the bromide anion in the solution is measured by any appropriate method. For example, a bromide specific ion probe can be used. The ion specific probe can be calibrated to read moles/liter.
3. The measured concentration of the bromide divided by the theoretical concentration times 100 equals the percent available for exchange.
4. Percent ionization as measured by this test of from 10% to 100% indicates a solvent in which the bromide anion is available for contact with the ion exchange resin. It is preferred that a percent ionization of 50%-100% is provided, and a percent ionization of 90% - 100% is even more preferred.

Another test that is useful for determining whether a solvent is one in which the bromide is available for contact with the ion exchange resin comprises:
1. Prepare a 1 molar solution of quat salt of a first anion in the solvent to be tested as described in the first step of the test described just above.
2. Contact the solution with a stoichiometric excess of an ion exchange resin in the form of the second anion at a temperature that is appropriate for the exchange by preparing a bed of the resin after regeneration to the form of the second anion and flowing the solution through the resin bed. A sample is taken from the first 5% by volume of the solution to exit the resin bed.
3. Measure the total amount of the first anion in the solution. The measuring technique must measure the amount of the first anion in any form.
4. If the concentration of the total amount of the first anion in the solution is less than 10% of the concentration of the first anion in the feed to the column, the solvent is one in which the first anion is available for contact with the ion exchange resin. For example, if the feed solution contains 5%, by weight bromide as bromine (whether present as an ion or in a salt), the solvent is one in which the first anion is available if the concentration of bromine in the solution is less than 0.1 x 5%, or less than 0.5%. Preferably, the solution will have less than 5%, and more preferably less than 1% of the concentration of the first anion in the feed to the column.

A preferred solvent for the present invention comprises a mixture of water and one or more polar organic co-solvents. As used herein, an "organic" co-solvent is a compound comprising carbon and hydrogen that is a liquid at room temperature. Also, as used herein, a "polar" co-solvent is a liquid at room temperature that has a dielectric constant that is greater than 4 at 25°C. Preferred polar co-solvents have a dielectric constant that is greater than 6 at 25°C, and more preferred co-solvents have a dielectric constant that is greater than 10 at 25°C.

It is preferred that the co-solvent also be a participating and volatile solvent. A co-solvent is a volatile solvent if it has a boiling point that is lower than the boiling point of pure water under the same conditions, or if it forms a constant boiling azeotrope that has such a boiling point. By way of example, when the tetraalkylammonium salt is a double-tailed molecule, such as a didecyldimethylammonium salt, such co-solvents have been found to facilitate solubilization of the double-tailed, molecule in an aqueous environment and allow for good equilibrium and good ion exchange. Examples of suitable organic co-solvents include several classes of compounds, such as:
alcohols, such as methanol, ethanol, isopropyl alcohol, propanol, butanol, isobutyl alcohol, C₁ - C₆ alcohols, C₁ - C₄ alcohols, and the like, with methanol being a preferred example;
polyalcohols, such as ethylene glycol, propylene glycol, and the like;
esters, such as ethyl acetate, propyl acetate, formates, and the like;
ethers, such as methyl tert-butyl ether, dioxane, glymes, and the like; and
carbonyl-containing solvents, such as acetone, acetaldehyde, and the like.

The polar organic co-solvent must exhibit some compatibility with water. It is preferred that any organic co-solvent that is used is one that is miscible with water.

For the solvents of the present method, it has been found that the ratios of the co-solvent to water are critical to the overall operative efficiency of the present ion exchange operation. Ranges from 10:90 up to 99:1 (co-solvent:water) are effective under the conditions/controls that are described herein, and depending upon the identity of the tetraalkylammonium compound. In general, it is preferred that the ratio of co-solvent : water, by weight, is within the range of from 50:50 to 99:1, 60:40 to 99:1 is more preferred, 70:30 to 98:2 is even more preferred, and 80:20 to 95:5 is yet more preferred.

It has generally been found that solvents having a higher ratio of co-solvent to water are preferred for quats having very hydrophobic alkyl substituent groups, *e.g.,* double tailed or twin tailed quats where the alkyl groups are C₁₀ - C₂₀, for example, while solvents having a lower ratio of co-solvent to water are preferred for quats having less hydrophobic alkyl substituent groups, *e.g.,* a (C₂ - C₆) alkyltrimethylammonium salt.

In one embodiment of the present invention, the solvent comprises a mixture of alcohol and water. It has been found to be preferred that when a mixture of alcohol and water is used as the solvent, the solvent comprises a mixture of a C₁ - C₆ alcohol and water in a ratio of from 10:90 to 99:1 by weight. Even more preferred is a solvent that comprises a mixture of a C₁ - C₄ alcohol and water in a ratio of from 50:50 to 99:1, 60:40 to 99:1 is more preferred, 70:30 to 98:2 is even more preferred, and 80:20 to 95:5 is yet more preferred. An example of a useful solvent for the present invention when the tetraalkylammonium salt is a didecyldimethylammonium salt is a mixture of methanol: water at a ratio of 85:15, by weight.

It should be understood that when a quat salt is added to a solvent that has two liquid components, the mixture becomes a ternary composition, with at least three major components, water, co-solvent, and quat salt. By way of example, a solution formed by adding 25% by weight of a quat salt to a solvent that comprised an 85:15 by weight mixture of methanol: water, would have a ternary composition, by weight, of 25:64:11, quat salt: methanol: water. When the quat salt is a tetraalkylammonium bromide salt, this is one example of a preferred solution for contact with an ion exchange resin in the present invention.

In the present method, the solution containing the quat bromide salt is contacted with an ion exchange resin in hydroxide form. While each and every active site on the resin need not be complexed with the stipulated anion, it is preferred that an ion exchange resin in hydroxide form have at least a majority of its active sites complexed with hydroxide. It is more preferred that at least 75%, even more preferred that at least 85%, and yet more preferred that at least 90% of the active sites on the resin are complexed with a hydroxide anion.

As will be apparent, during the anion exchange step of the present invention, the ion exchange resin will be donating hydroxide anions and gaining bromide anions, so that at any one time, both bromide and hydroxide anions will be present on the resin. When the resin has donated substantially all of the hydroxide anions and has substantially all of its active sites complexed with bromide anions, break through of the bromide anion will occur and the cycle of exchange will end. Therefore, at the start of an exchange cycle, the ion exchange resin is predominantly in the form of hydroxide anion, and at the end of an exchange cycle the ion exchange resin is predominantly in the form of the bromide anion. Prior to reuse or recycle, if that is desired, the resin must be regenerated back in the hydroxide form.

While almost any resin that will exchange anions can be used in the present invention, it is preferred that the resin is a (Type 2) strong base anion exchange resin. The resin can be either a gel-type resin or a macroreticular resin. In some applications, a gel-type resin is preferred, while a macroreticular resin is preferred in other applications. In certain systems, macroreticular resins appear to provide superior exchange. Examples of ion exchange resins that are useful in the present invention include certain Dowex® resins, such as Dowex® Marathon resins, Dowex ® Upcore resins, Dowex® Monosphere resins, Dowex® SBR resins, and the like, available from The Dow Chemical Co., Midland, MI; SBG, SBM, SBACR resins, and the like, available from Resintech, Inc., West Berlin, NJ; AG resins, available from Bio-Rad Laboratories, Inc., Hercules, CA; Type 2 resins available from QualiChem, Inc:, Salem, VA; Amberlite® IR and IRA resins, available from Rohn & Haas Company, Philadelphia, PA; Ionac® resins, available from Sybron Chemicals, at Lanxess Corporation, Pittsburgh, PA; and Spectra/Gel® anion exchange resins, available from Spectrum Chromatography, Houston, TX; among others.

The ion exchange resin that is used in the present invention should be prepared for use according to the directions provided by the manufacturer. These resins are commonly supplied in chloride form, and if the second anion in the present method is not chloride, then the resins must be converted to the second anion form before they can be used in the method. This is commonly done by contacting the resin in chloride form with a solution containing the second anion of the present process. The solution containing the second anion can be an acid, a salt, or a base, but it must be able to serve as a source for the second anion. For example, if the second anion in the present process is hydroxide, then new resin in chloride form can be contacted with a strong base, such as sodium hydroxide, to convert the chloride form resin into the hydroxide form resin.

The solution containing the tetraalkylammonium bromide salt can be contacted with the ion exchange resin in hydroxide form in any manner that is known in the art. The contact can be in batch mode or in continuous flow mode. When it is said that the contact can be in batch mode, it is meant that an amount of the ion exchange resin in hydroxide form is intermixed with an amount of the tetraalkylammonium bromide salt in the solvent and the mixture is permitted to remain in contact for some period of time - commonly until equilibrium is reached. The resin and solution can then be separated and the quat hydroxide salt can be recovered from the solution.

Commonly, when the present method is used at a commercial scale, it is preferred that the ion exchange resin is placed in a vessel, such as a column, to form a resin bed. The solution containing the tetraalkylammonium bromide salt is fed into the vessel containing the bed of the ion exchange resin in hydroxide form so that the solution passes through the resin bed during which time the bromide anions exchange with the hydroxide anions to form the tetraalkylammonium hydroxide salt and the ion exchange resin complexed with bromide. Due to selection of the proper solvent, the tetralkylammonium hydroxide salt remains in solution and can be separated from the resin. When the resin bed has become saturated with bromide anions, the solution exiting the bed will show an increase in the concentration of bromide. This point is termed the break through point and can signal the end of the feed step. At this point, the bed can be regenerated, as discussed below, to prepare it for the addition of more feed. The feed and regeneration steps comprises one cycle of bed operation.

When the ion exchange step is performed in a typical ion exchange column, the bed of ion exchange resin is contained in the column and the feed solution is fed to the column at either the top of the bed and removed at the bottom (downflow mode), or fed to the column at the bottom and removed at the top (upflow mode).

When the solution comprising the quat bromide salt in a solvent is fed to an ion exchange bed, the feed rate can be expressed in terms of the volume of the feed per unit of time per unit of ion exchange bed surface area, i.e., liter per minute per m² (gallons per minute per square foot) of bed surface area (l/min.m² (gpm/ft²)). This may also be termed the bed loading, or loading rate. In the present invention, the ion exchange step has been found to be successful when the loading rate is between 4.075 l/min.m² (0.1 gpm/ft²) and 203.73 l/min.m² (5 gpm/ft²), and a preferred loading rate is between 12.23 l/min.m² (0.3 gpm/ft²) and 142.61 l/min.m² (3.5 gpm/ft²), between 24.45 l/min.m² (0.6 gpm/ft²) and 101.88 l/min.m² (2.5 gpm/ft²) is even more preferred.

It has been found that a relationship exists between the concentration of the quat salt in the feed and the allowable loading rate, so that as the feed concentration is increased, the maximum allowable loading rate must be decreased in order that the product meet specifications for residual amounts of bromide. The loading rate and the feed concentration can be adjusted to maximize bed utilization efficiency. It has also been found that the composition of the solvent, as discussed above, is also a factor in arriving at an optimum ion exchange bed operation scheme.

The purpose of the ion exchange step of the present invention is the conversion of the tetraalkylammonium bromide salt to the tetraalkylammonium hydroxide salt. Although there is no particular minimum amount of conversion that must occur in order to carry out the present method, it is preferred that the degree of conversion be as high as practical. In an embodiment of the method is it preferred that at least 90 mol % of the tetraalkylammonium bromide salt is converted to the tetraalkylammonium hydroxide salt in the contacting step with the ion exchange resin, conversion of at least 99 mol % of the tetraalkylammonium bromide salt is more preferred, and conversion of at least 99.9 mol % of the tetraalkylammonium bromide salt to the tetraalkylammonium hydroxide salt is even more preferred.

Another purpose of the ion exchange step is to reduce the level of the bromide in the product solution to a level that is acceptable for the particular product that is being produced. For example, it is preferred that the solution containing the tetraalkylammonium hydroxide salt that is the product of the ion exchange step (the outflow of an ion exchange column) comprises less than 3000 ppm of bromide. When the concentration of the bromide anion is expressed in terms of parts per million (ppm), what is meant is the parts by weight of bromide per million parts by weight of final product (usually 50% by weight of the quat salt and 50% by weight solvent, usually water). When it is said that the particular solution comprises less than a certain amount of bromide, it is meant that the average concentration of bromide in the total amount of the solution that has been processed through the column since the last regeneration has less than the stated amount. In other words, product solution exiting a resin bed right after regeneration could have a lower level of bromide than product exiting the bed near break through. However, the average of the bromide in the total amount of the solution that had passed through the bed could be within the stated amount. In an embodiment of the present invention, it is preferred that the solution containing the tetraalkylammonium hydroxide salt after the ion exchange step comprises less than 1000 ppm of bromide, and less than 300 ppm is even more preferred. It is optionally preferred that the solution containing the tetraalkylammonium hydroxide salt after the ion exchange step comprises less than 300 ppm of bromide reported by weight as chloride.

The present method optionally includes the step of regenerating the ion exchange resin complexed with bromide and forming ion exchange resin in hydroxide form. To be of practical commercial utility, the ion exchange resin must be able to be regenerated over repeated cycles. For example, it is preferred that the ion exchange resin is regenerable on a daily, or more frequent, basis, with no significant losses in exchange efficiency over the span of a year of operation.

The ion exchange resin that is useful in the present method can be regenerated by any manner that is known in the art. Generally, the ion exchange resin can be regenerated by contacting it with an excess of a strong hydroxide base, such as sodium hydroxide. Although the regenerating compound can be in solution in any liquid, it is typical to regenerate the ion exchange resin with an aqueous solution of the compound. The sodium bromide salt solution that is a by-product of regeneration of a resin in bromide form can be sent to a recovery process, if desired, where sodium hydroxide and bromine can be recovered.

When sodium hydroxide is used to regenerate an ion exchange resin, it has been found to be preferred to contact the resin with a molar excess of the sodium hydroxide relative to the moles of bromide that are complexed with the resin at the time regeneration is begun. For example, if the resin is complexed with 1 mole of bromide, then it is preferred that from 2 to 10 moles of sodium hydroxide are contacted with the resin in order to successfully regenerate the resin into hydroxide form. It is more preferable that from 3 to 5 moles caustic per mole of bromide anion on the resin be used, and even more preferred that the molar ratio of caustic : bromide is between 4 and 4.5.

The inventors have found that the bed regeneration procedures for the ion exchange resin system have a significant effect on the economics of the method of the invention. Factors such as the concentration of the base in the regenerating solution, the flow rate of the regenerating solution, and the total amount of base that is used relative to the amount of first anion that must be displaced from the resin, have all been found to be useful for control of the efficiency of the regeneration process. For example, FIG. 2 shows a graph of the bromide concentration profile taken at the mid-point of an ion exchange bed. A 20% quat bromide stream was fed to this bed and converted to hydroxide form. The bed was then regenerated with 4 equivalents of sodium hydroxide per bromide equivalent fed. Runs using 4% and then 8% sodium hydroxide for regeneration were completed. The breakthrough curve following the regenerations with 4% sodium hydroxide was sharper than the curve which follows regeneration with 8% sodium hydroxide, and occurs later during the feed cycle. This indicates regeneration with more dilute caustic can increase effective bed capacity, or could be used to reduce the amount of caustic required for regeneration at an equivalent capacity demonstrated with 8% regenerations.

When the first anion is a halide and the second anion is hydroxide, it is preferred that the regeneration of the ion exchange resin in halide form comprises contacting the resin in an ion exchange resin bed with an aqueous solution comprising at least 1% - 25% sodium hydroxide by weight at a rate of at least 4.075 l per minute per m² (0.1 gallons per minute per square foot) of ion exchange bed surface area ((l/min.m²) (gpm/ft²)). It has been found that dilute solutions of sodium hydroxide make the most effective use of hydroxide equivalents, but the volumes of dilute streams often result in higher operating costs. As an economic balance, hydroxide solution concentration ranges between 2% -12%, by weight, are preferred, and concentrations between 4% - 8%, by weight, are more preferred.

Furthermore, it has been found to be preferred that the aqueous solution of sodium hydroxide is contacted with the ion exchange bed at a rate of at least 12.23 l/min.m² (0.3 gpm/ft²), and to be more preferred that the aqueous solution of sodium hydroxide is contacted with the ion exchange bed at a rate between 20.375 and 101.88 l/min.m² (0.5 and 2.5 gpm/ft²), and even more preferred that the aqueous solution of sodium hydroxide is contacted with the ion exchange bed at a rate between 24.45 and 61.125 l/min.m² (0.6 and 1.5 gpm/ft²).

Although the feed step and the regeneration of the ion exchange bed can proceed in either upflow or downflow mode, when the bed is in a vessel or column it has been found to be preferred that the regenerating solution is contacted with the ion exchange bed in a mode that is opposite to that used for the feed step. By way of example, when the bed is fed in upflow mode, then regeneration in downflow mode is preferred, and vice versa.

After regeneration of the ion exchange resin, the present method can optionally include recycling all or a part of the regenerated ion exchange resin in hydroxide form for use in contacting the solution containing the tetraalkylammonium bromide salt. Of course, this can recur a number of times.

It should be noted that the ion exchange resin can be rinsed, eluted, or purged with a liquid, such as water or the solvent, between any of the steps of the present method.

In the present method, the quaternary tetraalkylammonium hydroxide salt is converted to the quaternary tetraalkylammonium salt of a second (or selected) anion. While almost any anion can be used as the second anion, it is common to select a second anion that provides desirable properties to the final product quat salt. It is preferred that the second anion is different from either bromide or hydroxide. By way of example, useful anions that can act as the second anion of the present invention include halide, hydroxide, borate, formate, carboxylates, carbonate, bicarbonate, sulfate, bisulfate, sulfite, sulfonate, phosphate, phosphonate, nitrate, chlorate, acetate, and mixtures thereof.

The quat hydroxide salt can be converted to the quat salt of the second anion by any method that is known in the art. However, two methods have been shown to be particularly useful for this step. In the first method, the step of converting the quaternary tetraalkylammonium hydroxide salt to the quaternary tetraalkylammonium salt of the second anion comprises contacting the solution of the tetraalkylammonium hydroxide salt with an ion exchange resin in the form of the second anion and forming a tetraalkylammonium salt of the second anion in solution and the ion exchange resin in hydroxide form.

In the present invention, the optional ion exchange step that is used to convert a quat hydroxide salt to a quat salt of a second anion can be referred to herein as the second ion exchange step. This second ion exchange step preferably is carried out in a manner that is similar to the ion exchange step for the conversion of the quat bromide to the quat hydroxide that is discussed above, except that where the discussion above refers to the quat bromide salt, or the tetraalkylammonium bromide salt, in this second ion exchange step the quat hydroxide salt, or the tetraalkylammonium hydroxide salt, respectively, would be substituted, and where the discussion above refers to the quat hydroxide salt, or the tetraalkylammonium hydroxide salt, in this second ion exchange step the quat salt of a second anion, or the tetraalkylammonium salt of a second anion, respectively, would be substituted. The selection of the solvent would be carried out in a similar way and with similar methods of determination. The resin used for the second ion exchange step could be the same as was used in the first step, or it could be different. Likewise, the manner of operation of a resin bed to carry out the second ion exchange is similar to that described above. When the spent resin bed is regenerated for the second ion exchange step, the spent resin is in hydroxide form, and it must be contacted with one or more regenerating solutions that can act as a source for the second anion in order to return the resin to the desired form of an ion exchange resin in the form of a second anion so that it can be recycled or reused.

Although the second ion exchange step can be operated under conditions that cause the exchange of any fraction of the hydroxide anions for the second anions, it is preferred that at least 90 mol % of the tetraalkylammonium hydroxide salt is converted to the tetraalkylammonium salt of the second anion, and more preferred that at least 99 mol % of the tetraalkylammonium hydroxide salt is converted to the tetraalkylammonium salt of the second anion, and even more preferred that at least 99.9 mol % of the tetraalkylammonium hydroxide salt is converted to the tetraalkylammonium salt of the second anion.

When the second ion exchange step (*i.e*., conversion of the quat hydroxide salt to the quat salt of a second anion by contact with an ion exchange resin) is included in the present method, the method can optionally include regenerating the ion exchange resin in hydroxide form and forming the ion exchange resin in the form of the second anion. As described above, this regeneration can comprise contacting the resin with a solution comprising the second anion and forming ion exchange resin in the form of the second anion.

In a particularly useful embodiment of the present method, the second anion is carbonate or a mixture of carbonate and bicarbonate.

An alternative method that has been found to be useful for the conversion of the quaternary tetraalkylammonium hydroxide salt to the quaternary tetraalkylammonium salt of the second anion comprises contacting the tetraalkylammonium hydroxide salt with an acid of the second anion and converting the tetraalkylammonium hydroxide salt to a tetraalkylammonium salt of the second anion.

In this embodiment, the acid of the second anion comprises an acid having as an anion at least one anion except for bromide that is selected from the group consisting of halide, borate, formate, carboxylates, carbonate, bicarbonate, sulfate, bisulfate, sulfite, sulfonate, phosphate, phosphonate, nitrate, chlorate, acetate, and mixtures thereof. The contact between the acid and the ion exchange resin in hydroxide form then becomes an acid/base reaction that leaves the resin in the form of the second anion, and forms water from the hydrogen and the hydroxide ions.

By way of example, the acid can be carbonic acid that is formed by contacting carbon dioxide with an aqueous solution. The carbonic acid in aqueous solution can form carbonate anions or a mixture of carbonate and bicarbonate anions. When carbonic acid is the acid that is contacted with the quat hydroxide salt, a quat carbonate salt, or a mixture of quat carbonate and quat bicarbonate salts are formed. As more carbon dioxide is added to the solution, successively more bicarbonates are formed as carbonates are changed to bicarbonates. Therefore, it is possible to control the ratio of quart carbonate-to-quat bicarbonate by controlling the amount of carbon dioxide that is contacted with the solution containing the quat hydroxide.

Although the step of contacting the tetraalkylammonium hydroxide salt with an acid of the second anion and converting at least a portion of the tetraalkylammonium hydroxide salt to a tetraalkylammonium salt of the second anion can be carried out in any type of equipment where an acid can be intermixed with the quat hydroxide salt, it has been found to be useful to perform this step in a reactive distillation column.

In a reactive distillation column, as illustrated in FIG.3, the solution comprising the tetraalkylammonium hydroxide salt (Q-OH) is fed to the column as is the carbon dioxide (CO2). The carbon dioxide is fed to the column at a point where it will flow up the column thereby forming carbonic acid which reacts with the tetraalkylammonium hydroxide salt and forms a tetraalkylammonium carbonate and/or bicarbonate salt which exits the bottom of the column in an aqueous solution. The co-solvent is stripped from the feed solution and exits the top of the column. Further information about the operation of reactive distillation separation in general can be found in Sundmacher, K. and Kienle, A., Reactive Distillation : Status and Future Directions, Wiley-VCH, (2003).

The use of a reactive distillation column has been found to be useful when the tetraalkylammonium salt of a second anion comprises didecyldimethylammonium carbonate and didecyldimethylammonium bicarbonate. Furthermore, this embodiment can be used advantageously when the tetraalkylammonium salt of a second anion comprises didecyldimethylammonium carbonate and didecyldimethylammonium bicarbonate in a molar ratio of 10:90.

The present invention also encompasses compositions containing at least one quaternary ammonium compound made according to the methods described herein and optionally other active or inert ingredients. Specific materials that can be produced with the quaternary ammonium compounds made according to the methods described herein include, but are not limited to, wood preservatives, surfactants, insecticides, biocides, disinfectants, textile chemicals, oilfield chemicals, mining chemicals, shampoos, laundry detergents, cosmetics, wetting agents, polyurethane foam catalysis, and epoxy curing agents.

In some embodiments, the final quaternary ammonium composition provided by the present invention comprises less than 100 ppm of any contaminating halide. As used herein, the terms "contaminating halide" refer to any halide that remains after a final ion-exchange reaction. In some embodiments, the quaternary ammonium composition comprises less than 10 ppm of contaminating halide, and in other embodiments, the composition comprises less than 5 ppm of any contaminating halide.

In other aspects of the present invention, a finished quat can be prepared that has only a single species of quat-anion. In some instances, the present invention also provides a highly purified quaternary ammonium composition produced according to the methods described herein that contains nearly 99% or higher of a single type of quat counter-ion (anion). For example, when the bicarbonate anion form of the quat is produced according to the methods herein, the resulting quat composition contains 99% or higher of solely the bicarbonate anion and less than 1 % of any other anion species. In still other embodiments, the quat composition contains 95% or higher of a single type of quat anion and, in some embodiments, it contains 90% or higher of a single type of quat anion.

A composition containing a quaternary ammonium compound produced according to the methods described herein may also be formulated as a wood preservative composition by itself or formulated in combination with compounds such as copper (e.g., copper oxide). Such a wood preservative composition may be applied to any wood substrate, such as any hard wood or soft wood, to prevent or lessen degradation and staining. Typically, for preventing or controlling staining and mold, the wood preservative composition is applied to green wood. The term "green" as used herein is defined as freshly cut, unseasoned, or the like. Examples of suitable wood substrates include, but are not limited to, maple, oak, birch, cherry, fir, and the like. The wood preservative composition may be applied to any wood substrate which is to be pressure treated. In one embodiment, the wood substrate is a soft wood, such as a pine, fir, or hemlock. Suitable pine wood substrates include, but are not limited to, southern yellow pine and ponderosa pine. In certain aspects of the present invention, the wood substrate is southern yellow pine.

Methods of applying the wood preservative composition include, but are not limited to, spraying, soaking, immersing, vacuum impregnation, pressure treatment, brushing, and the like. In one embodiment, the wood substrate is immersed in the wood preservative composition of the present invention or the substrate is pressure treated with the composition.

A wood substrate containing the wood preservative composition generally contains from 0.1 to 5% by weight of the wood preservative composition made with the quaternary ammonium compounds produced according to the methods described herein, and in some embodiments, from 0.25 to 3% by weight, and in other embodiments, from 0.5 to 2% by weight, based upon 100% total weight of preserved wood substrate. In other embodiments, the wood preservative composition described herein is applied to the wood substrate at between 1.6 and 40 g of preservative per liter (0.1 and 2.5 pounds of preservative per cubic foot) of board length, and in some embodiments, it is applied at 4.16 g/l (0.26 pounds/ft³).

In certain aspects of the present invention, the quaternary ammonium compounds produced as detailed herein are used in the preparation of alkaline copper quat (ACQ)-type wood preservatives. Such preservatives contain copper (II) ions, carbonate and/or biocarbonate anions, and the quaternary ammonium compound. The typical ratio of copper expressed as copper oxide to quat is 2:1 1 by weight and the ratio of carbonate expressed as C0₂ or HC0₃ to CuO is 0.65:1 by weight.

In certain embodiments, such ACQ-type wood preservatives have the following compositions: 66.7% copper oxide (copper) and 33.3% quat. The compositions are, in certain embodiments, first dissolved in ethanolamine or ammonia to give aqueous solutions having a pH of from 8.5 to 11.5. When ethanolamine is used, the weight of ethanolamine in treating solutions is usually 2.75 times the weight of copper oxide and when ammonia is used, the weight of the ammonia in treating solutions is usually at least 1.0 times the weight of copper oxide. The amount of carbonate anion is typically at least 0.69 times the amount of copper oxide.

The following examples describe various embodiments of the present invention. Other embodiments within the scope hereof will be apparent to one skilled in the art from consideration of the specification or practice of the invention as disclosed herein. It is intended that the specification, together with the examples, be considered to be exemplary only, with the scope and spirit of the invention described herein. In the examples, all percentages are given on a weight basis unless otherwise indicated.

### GENERAL PROCEDURES

The general scheme of one embodiment of the present invention provides a method for the production of a quaternary ammonium compound having the structure according to Formula I: 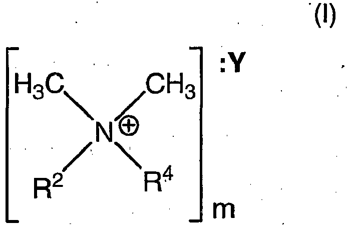 wherein:
R² is substituted or unsubstituted alkyl or substituted or unsubstituted alkenyl, which, if substituted, has one or more substituent groups selected from aryl, heterocyclyl, hydroxyl, ester, benzyl, carboxyl, halo, nitro, cyano, alkoxy or oxo, or R² is 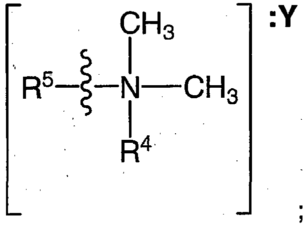
R⁴ is substituted or unsubstituted alkyl or substituted or unsubstituted alkenyl, which, if substituted, has one or more substituent groups selected from aryl, heterocyclyl, hydroxyl, ester, benzyl, carboxyl, halo, nitro, cyano, alkoxy or oxo;
R⁵ is substituted or unsubstituted alkyl or substituted or unsubstituted alkenyl, which, if substituted, has one or more substituent groups selected from aryl, heterocyclyl, hydroxyl, ester, benzyl, carboxyl, halo, nitro, cyano, alkoxy or oxo;
**Y** is an anion having a valence of -1, -2 or -3, and is selected from the group consisting of bicarbonate, carbonate, carboxylate, hydroxide, phosphate, chloride, sulfate, oxalate, nitrate, fluoride, bisulfate, acetate, formate, borate, and mixtures thereof;
m is 1, 2 or 3; and
comprising the steps of:
a) reacting an alkyldimethylamine compound having the structure according to Formula II: 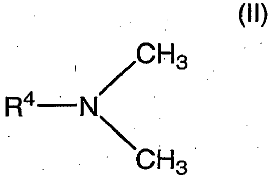 wherein R⁴ is defined as above;
   with an alkyl halide compound having the structure:

   R²-Z or Z-R²-Z;

   wherein R² is defined as above;
   **Z** is a halide that is selected from the group consisting of bromide, chloride, and iodide;
   to form a quaternary intermediate compound having the structure according to Formula III: 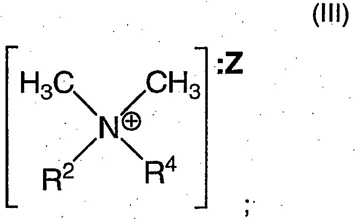
b) solubilizing the quaternary intermediate compound in a first mixture of water and alcohol; and
c) exchanging **Z** complexed with the quaternary intermediate compound with **Y** by contacting the solubilized quaternary intermediate compound with an ion-exchange resin which comprises **Y**, wherein the exchange of **Z** with **Y** forms the quaternary ammonium compound having the structure according to Formula I.

As used herein, when the term "alkyl" is used, either alone or within other terms such as "haloalkyl" and "alkylsulfonyl" it embraces linear or branched radicals composed of carbon atoms. Unless otherwise noted, such radicals preferably contain from 2 to 20 carbon atoms, preferably from 6 to 18 carbon atoms, and more preferably from 8 to 12 carbon atoms. The number of carbon atoms can also be expressed as "C₁-C5", for example. Examples of such radicals include methyl, ethyl, n-propyl, isopropyl, n-butyl, isobutyl, sec-butyl, tert-butyl, pentyl, isoamyl, hexyl, octyl and the like. When an alkyl radical, such as, for example," butyl" is used, it is intended that all primary, secondary and tertiary forms of the alkyl radical are encompassed by the term, including n-butyl, t-butyl, isobutyl and sec-butyl.

The term "alkenyl" refers to an unsaturated, acyclic hydrocarbon radical, linear or branched, in so much as it contains one double bond. Unless otherwise noted, such radicals preferably contain from 2 to 20 carbon atoms, preferably from 6 to 18 carbon atoms, and more preferably from 8 to 12 carbon atoms. Examples of suitable alkenyl radicals include propenyl, 2-chloropropylenyl, buten-1yl, isobutenyl, penten-1yl, 2-methylbuten-1-yl, 3-methylbuten-1-yl, hexen-1-yl, 3-hydroxyhexen-1-yl, hepten-1-yl, octen-1-yl, and the like.

The term "alkoxy" includes linear or branched oxy-containing radicals, each of which has, unless otherwise noted, alkyl portions of 1 to 6 carbon atoms, preferably 1 to 4 carbon atoms, such as methoxy, ethoxy, propoxy, isopropoxy, isobutoxy radicals, and the like.

The terms "hydrido", "-H", or "hydrogen", denote a single hydrogen atom (H). This hydrido radical may be attached, for example, to an oxygen atom to form a hydroxyl radical, or two hydrido radicals may be attached to a carbon atom to form a methylene (-CH₂ -) radical.

The term "aryl", alone or in combination, means a carbocyclic aromatic system containing carbon rings wherein such rings may be attached together in a pendent manner or may be fused. The term "aryl" embraces aromatic radicals such as phenyl, naphthyl, tetrahydronapthyl, indane, and biphenyl.

The term "oxo" means a single double-bonded oxygen.

In the naming of substituent groups for general chemical structures, the naming of the chemical components of the group is typically from the terminal group-toward the parent compound unless otherwise noted, as discussed below. In other words, the outermost chemical structure is named first, followed by the next structure in line, followed by the next, etc. until the structure that is connected to the parent structure is named. Substituent groups may also be named by reference to one or more "R" groups. The structure shown above would be included in a description, such as, "-C₁-C₆-alkyl-COR^{u}, where R^{u} is defined to include -NH-C₁-C₄-alkylaryl-R^{y}, and where R^{y} is defined to include halo. In this scheme, atoms having an "R" group are shown with the "R" group being the terminal group (*i.e*., farthest from the parent). In a term such as "C(R^{x})₂", it should be understood that the two R^{x} groups can be the same, or they can be different if R^{x} is defined as having more than one possible identity. Finally, the bonds having wavy lines through them represent the parent structure to which a particular structure is attached.

Also considered herein is a method for the production of a quaternary ammonium compound having the structure according to Formula I: 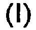 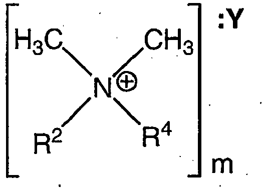 wherein:
R² is substituted or unsubstituted alkyl or substituted or unsubstituted alkenyl, which, if substituted, has one or more substituent groups selected from aryl, heterocyclyl, hydroxyl, ester, benzyl, carboxyl, halo, nitro, cyano, alkoxy or oxo;
R⁴ is substituted or unsubstituted alkyl or substituted or unsubstituted alkenyl, which, if substituted, has one or more substituent groups selected from aryl, heterocyclyl, hydroxyl, ester, benzyl, carboxyl, halo, nitro, cyano, alkoxy or oxo;
Y is an anion having a valence of -1, -2 or -3, and is selected from the group consisting of bicarbonate, carbonate, carboxylate, hydroxide, phosphate, chloride, sulfate, oxalate, nitrate, fluoride, bisulfate, acetate, formate, borate; and mixtures thereof;
m is 1, 2 or 3; and
comprising the steps of:
a) reacting an alkyldimethylamine compound having the structure according to Formula II: 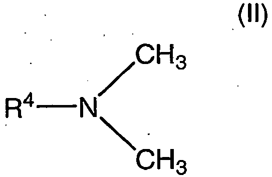 wherein R⁴ is defined as above;
   with an alkyl halide compound having the structure:

   R²-Z;

   wherein R² is defined as above;
   **Z** is a halide that is selected from the group consisting of bromide, chloride, and iodide;
   to form a quaternary intermediate compound having the structure according to Formula III: 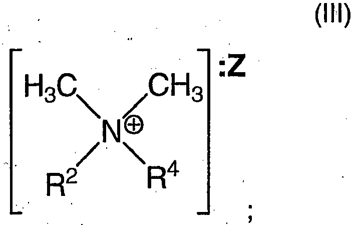
b) solubilizing the quaternary intermediate compound in a first mixture of water and alcohol; and
c) exchanging **Z** complexed with the quaternary intermediate compound with **Y** by contacting the solubilized quaternary intermediate compound with an ion-exchange resin which comprises **Y**, wherein the exchange of **Z** with **Y** forms the quaternary ammonium compound having the structure according to Formula I.

In another embodiment, the present invention also provides a method for the production of a quaternary ammonium compound having the structure according to Formula I: 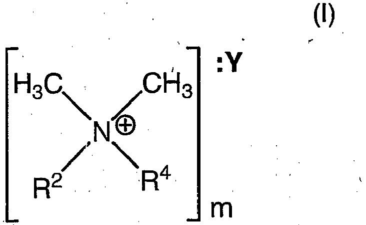 wherein:
R² is substituted or unsubstituted alkyl, which, if substituted, has one or more substituent groups selected from aryl, heterocyclyl, hydroxyl, ester, benzyl, carboxyl, halo, nitro, cyano, alkoxy or oxo;
R⁴ is substituted or unsubstituted alkyl, which, if substituted, has one or more substituent groups selected from aryl, heterocyclyl, hydroxyl, ester, benzyl, carboxyl, halo, nitro, cyano, alkoxy or oxo;
**Y** is an anion having a valence of -1, -2 or -3, and is selected from the group consisting of bicarbonate, carbonate, carboxylate, hydroxide, phosphate, chloride, sulfate, oxalate, nitrate, fluoride, bisulfate, acetate, formate, borate, and mixtures thereof;
mi s 1, 2 or 3; and
comprising the steps of:
a) reacting an alkyldimethylamine compound having the structure according to Formula II: 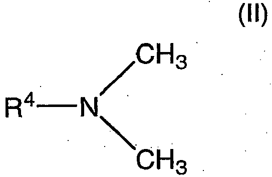 wherein R⁴ is defined as above;
   with an alkyl halide compound having the structure:

   R²-Z;

   wherein R² is defined as above;
   **Z** is a halide that is selected from the group consisting of bromide, chloride, and iodide;
   to form a quaternary intermediate compound having the structure according to Formula III: 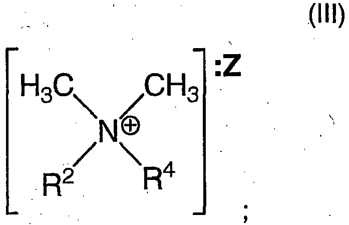
b) solubilizing the quaternary intermediate compound in a first mixture of water and alcohol; and
c) exchanging **Z** complexed with the quaternary intermediate compound with **Y** by contacting the solubilized quaternary intermediate compound with an ion-exchange resin which comprises **Y**, wherein the exchange of **Z** with **Y** forms the quaternary ammonium compound having the structure according to Formula I.

In another embodiment, the present invention also provides a method for the production of a quaternary ammonium compound having the structure according to Formula I: 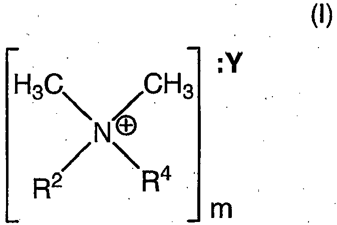 wherein:
R² and R⁴ are unsubstituted alkyl;
**Y** is an anion having a valence of -1, -2 or -3, and is selected from the group consisting of bicarbonate, carbonate, carboxylate, hydroxide, phosphate, chloride, sulfate, oxalate, nitrate, fluoride, bisulfate, acetate, formate, borate, and mixtures thereof;
m is 1, 2 or 3; and
comprising the steps of:
a) reacting an alkyldimethylamine compound having the structure according to Formula II: 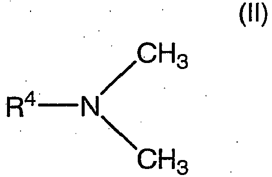 wherein R⁴ is defined as above;
   with an alkyl halide compound having the structure:

   R²-Z;

   wherein R² is defined as above;
   **Z** is a halide that is selected from the group consisting of bromide, chloride, and iodide;
   to form a quaternary intermediate compound having the structure according to Formula III: 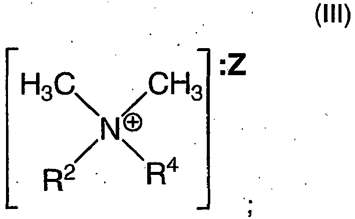
b) solubilizing the quaternary intermediate compound in a first mixture of water and alcohol; and
c) exchanging **Z** complexed with the quaternary intermediate compound with **Y** by contacting the solubilized quaternary intermediate compound with an ion-exchange resin which comprises **Y**, wherein the exchange of **Z** with **Y** forms the quaternary ammonium compound having the structure according to Formula I.

In another embodiment, the present invention also provides a method for the production of a quaternary ammonium compound having the structure according to Formula I: 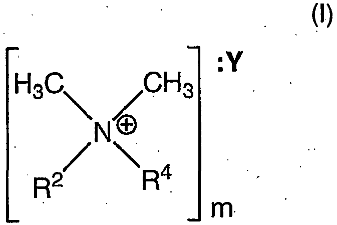 wherein:
R² and R⁴ are unsubstituted C₈-C₁₈ alkyl;
**Y** is an anion that is selected from the group consisting of bicarbonate, carbonate, carboxylate, hydroxide, phosphate, chloride, sulfate, oxalate, nitrate, fluoride, bisulfate, acetate, formate, borate, and mixtures thereof;
m is 1, 2 or 3; and
comprising the steps of:
a) reacting a C₈-C₁₈ alkyldimethylamine compound having the structure according to Formula II: 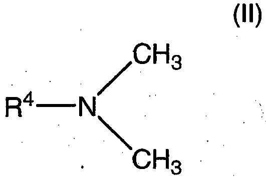 wherein R⁴ is defined as above;
   with an alkyl halide compound having the structure:

   R²-Z;

   wherein R² is defined as above;
   **Z** is bromide;
   to form a quaternary intermediate compound having the structure according to Formula III: 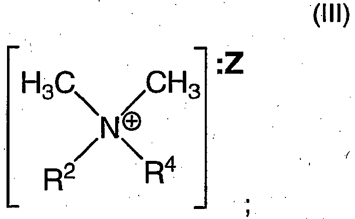
b) solubilizing the quaternary intermediate compound in a first mixture of water and alcohol; and
c) exchanging **Z** complexed with the quaternary intermediate compound with **Y** by contacting the solubilized quaternary intermediate compound with an ion-exchange resin which comprises **Y**, wherein the exchange of **Z** with **Y** forms the quaternary ammonium compound having the structure according to Formula I.

In another embodiment, the present invention also provides a method for the production of a quaternary ammonium compound having the structure according to Formula I: 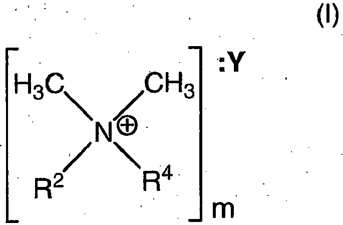 wherein:
R² and R⁴ are unsubstituted C₈-C₁₈ alkyl;
**Y** is an anion that is selected from the group consisting of bicarbonate, carbonate, hydroxide, chloride, and mixtures thereof;
m is 1 or 2; and
comprising the steps of:
a) reacting a C₈-C₁₈ alkyldimethylamine compound having the structure according to Formula II: 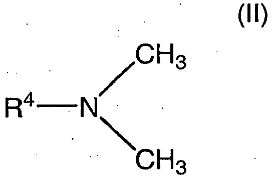 wherein R⁴ is defined as above;
   with an alkyl halide compound having the structure:

   R²-Z;

   wherein R² is defined as above;
   **Z** is bromide;
   to form a quaternary intermediate compound having the structure according to Formula III: 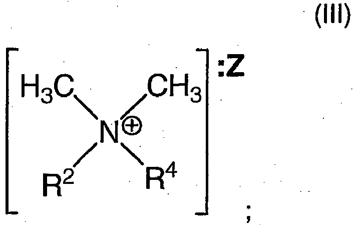
b) solubilizing the quaternary intermediate compound in a first mixture of water and alcohol; and
c) exchanging **Z** complexed with the quaternary intermediate compound with **Y** by contacting the solubilized quaternary intermediate compound with an ion-exchange resin which comprises **Y**, wherein the exchange of **Z** with **Y** forms the quaternary ammonium compound having the structure according to Formula I.

In another embodiment, the present invention also provides a method for the production of a quaternary ammonium compound having the structure according to Formula I: 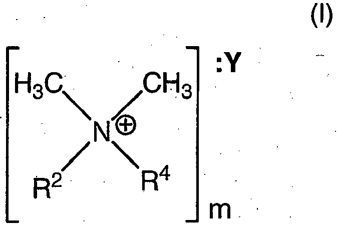 wherein:
R² and R⁴ are unsubstituted C₁₀ alkyl;
**Y** is an anion that is selected from the group consisting of bicarbonate, carbonate, and mixtures thereof;
m is 1 or 2; and
comprising the steps of:
a) reacting a C₁₀ alkyldimethylamine compound having the structure according to Formula II: 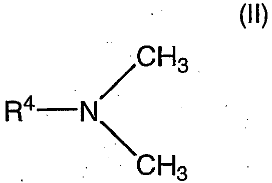 wherein R⁴ is defined as above;
   with an alkyl halide compound having the structure:

   R²-Z;

   wherein R² is defined as above;
   **Z** is bromide;
   to form a quaternary intermediate compound having the structure according to Formula III: 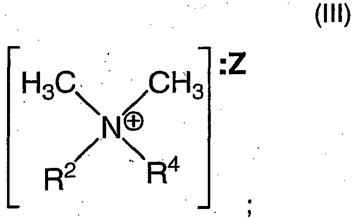
b) solubilizing the quaternary intermediate compound in a first mixture of water and alcohol; and
c) exchanging **Z** complexed with the quaternary intermediate compound with **Y** by contacting the solubilized quaternary intermediate compound with an ion-exchange resin which comprises **Y**, wherein the exchange of **Z** with **Y** forms the quaternary ammonium compound having the structure according to Formula I.

### EXAMPLE 1

This example demonstrates a synthesis reaction for the production of a quaternary ammonium intermediate compound having a bromide counter-ion.

A 1-liter, creased, 4-necked round bottom Pyrex flask equipped with mechanical stirrer, 250-milliliter (ml) addition funnel, temperature probe, heating mantle, and water-cooled total reflux condenser was used. The flask was initially charged with 200 grams (1.08 mole) of ADMA-1 0 (decyldimethylamine, available from Albemarle Corporation, Baton Rouge, LA), and 239 grams (1.08 mole) decyl bromide was placed in the addition funnel. The stirrer was turned on and the reactor was heated to 65°C. The decyl bromide was added dropwise to the ADMA-10 as the temperature of the reactor was allowed to rise from 65°C to 142°C. The addition funnel was then charged with 110 grams of methanol, and the methanol was added dropwise to the sotution as the temperature of the reactor was allowed to fall to 90°C. After the entire volume of the methanol was added to the reaction mixture, the heat and stirring were ceased and the intermediate quaternary ammonium (quat-Br) solution was allowed to cool.

### EXAMPLES 2 - 28.

These examples illustrate the production of dialkyldimethylammonium halide salts with varying reaction conditions and different reactants.

A number of dialkyldimethylammonium halide salts were produced according to the reaction methods described in Example1, except that different concentrations of reactants were used, the reactions were carried out in different sized vessels, the alkyl halide was added to the trialkylamine at different rates and at different times during the reaction, and the amount and type of solvent that was added at the end of the reaction was varied. The conditions of each run along with a number of parameters of the products that were produced are shown in Table 1.

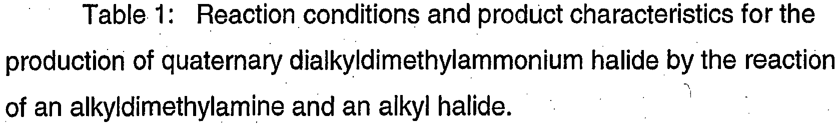

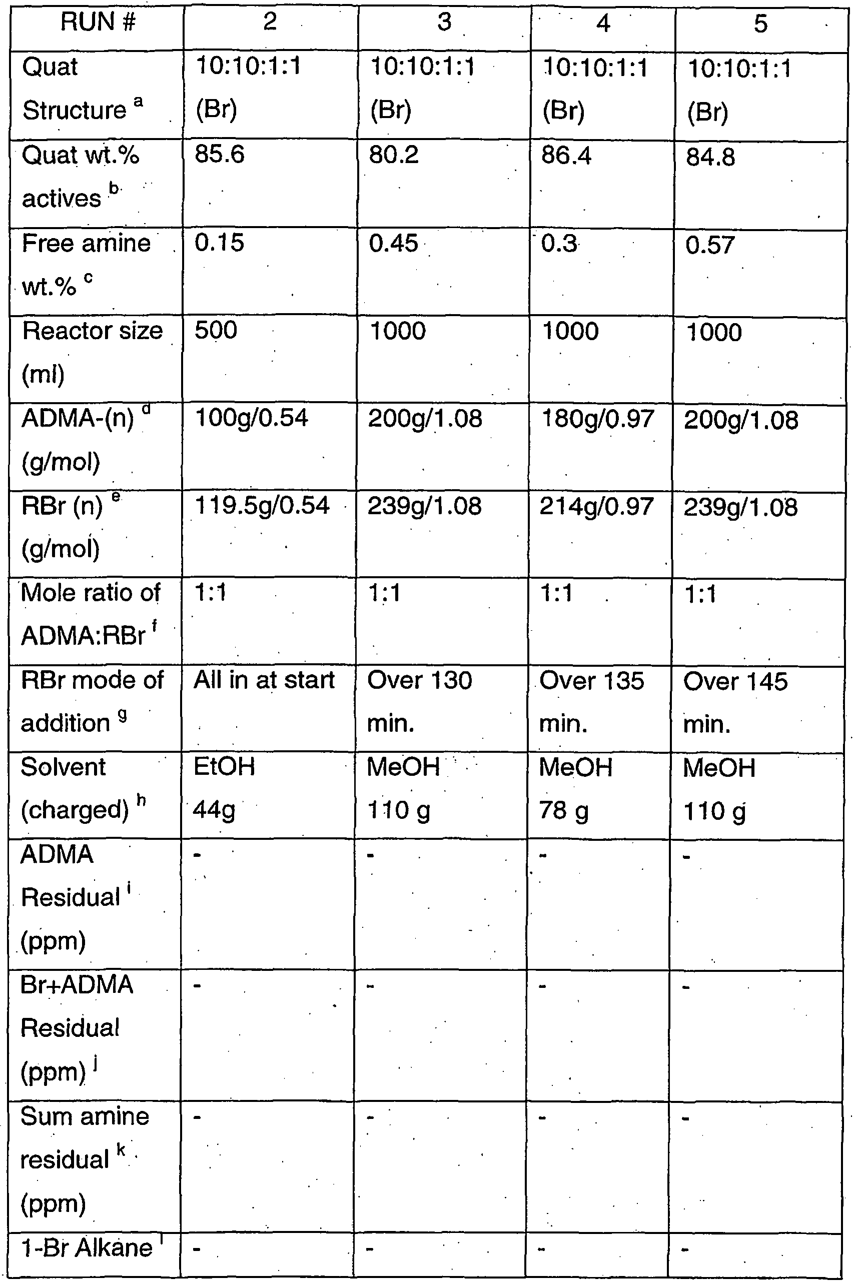

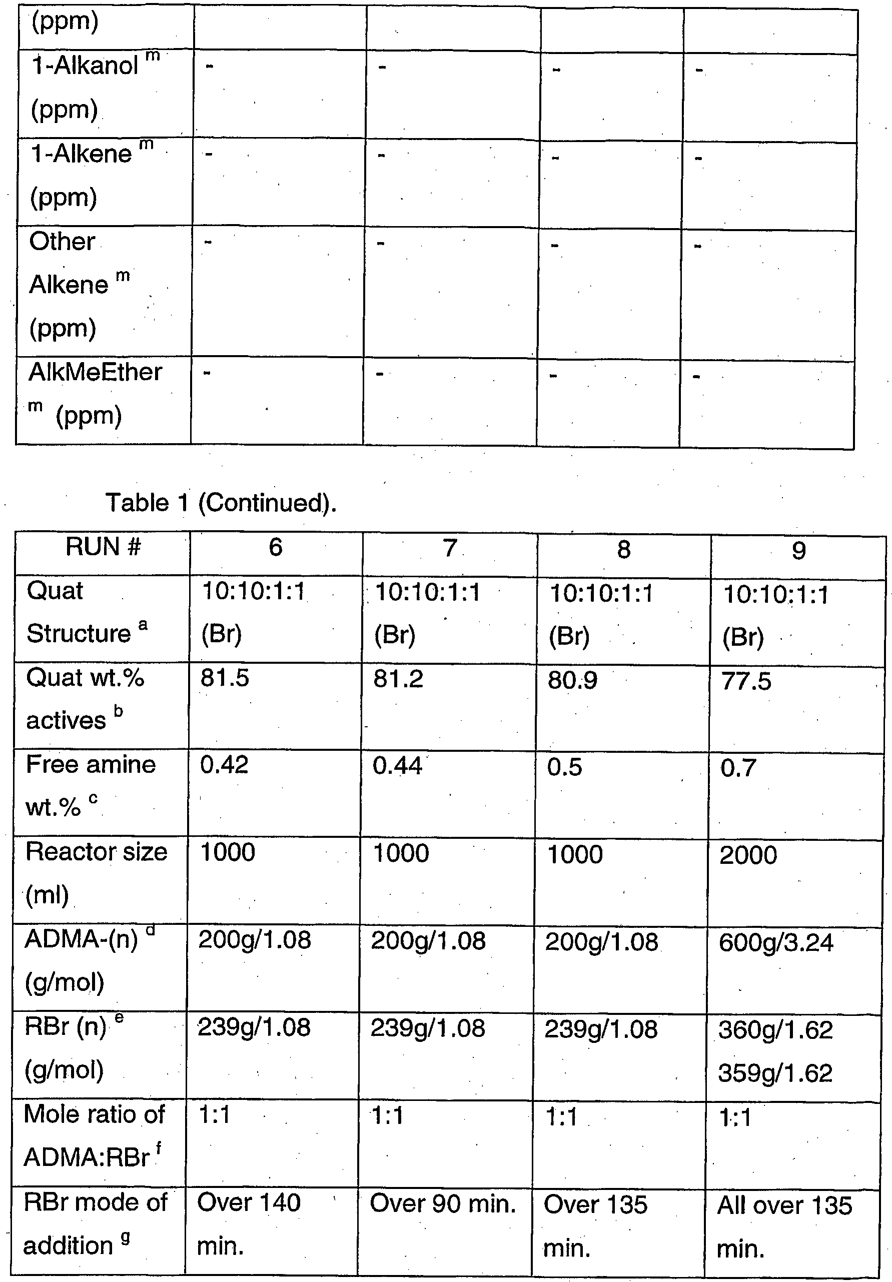

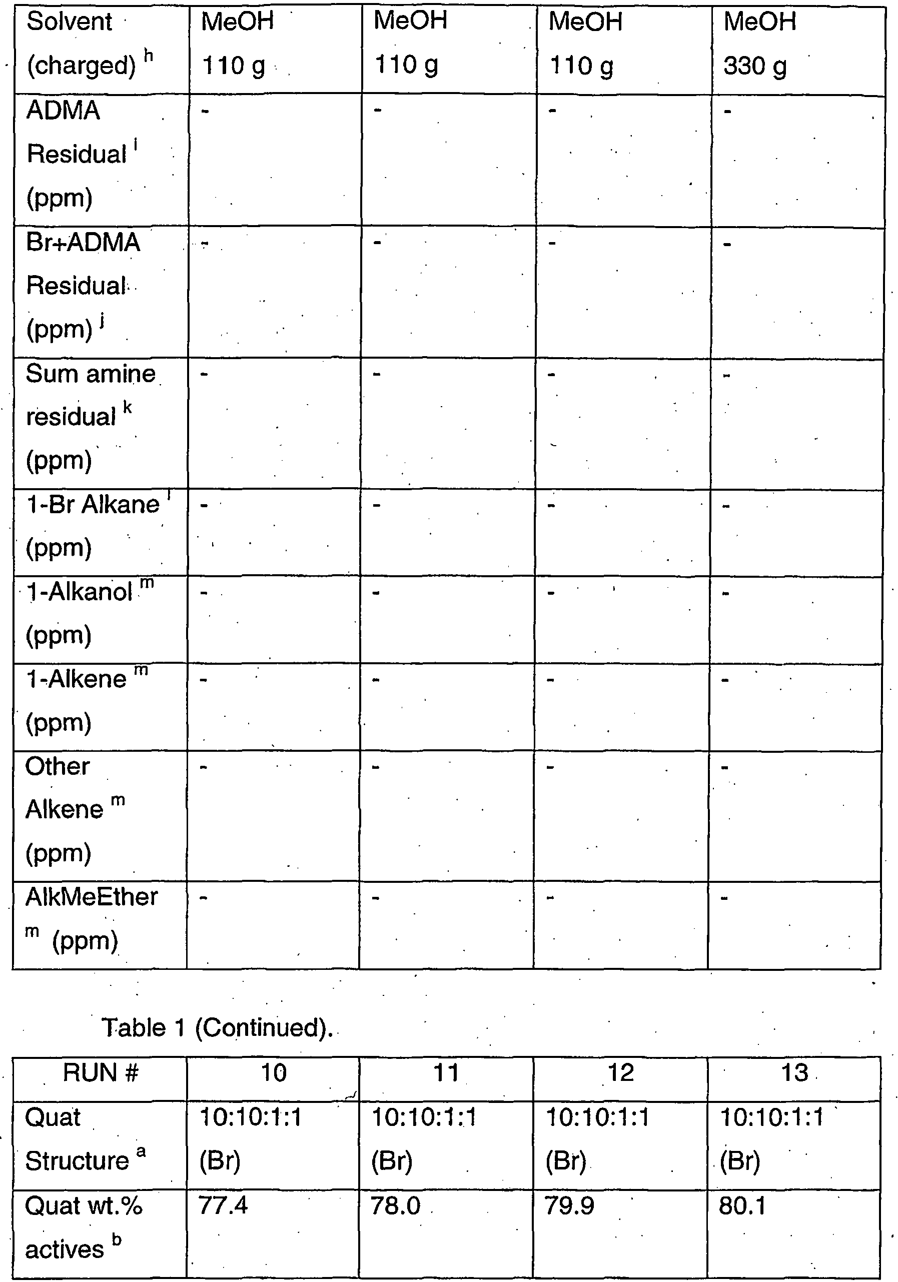

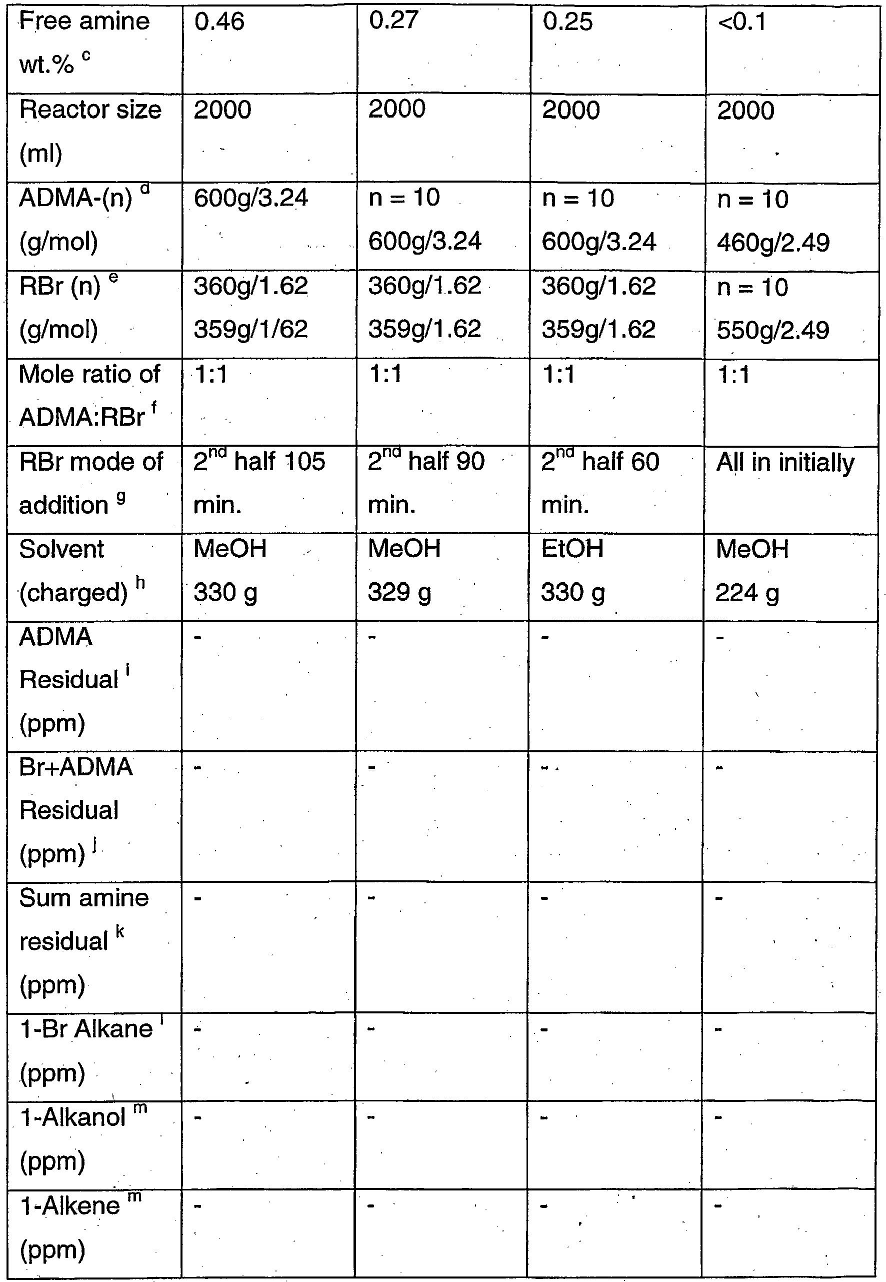

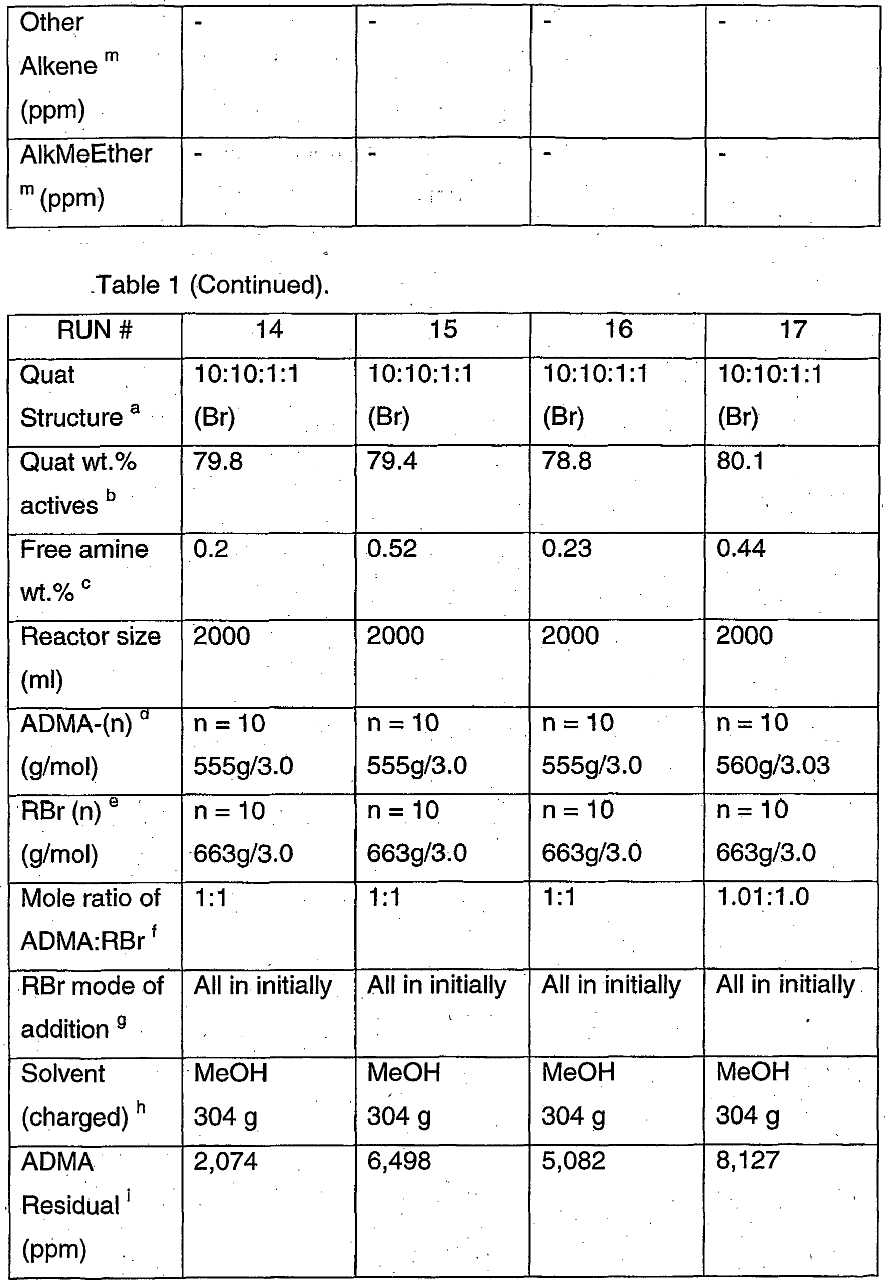

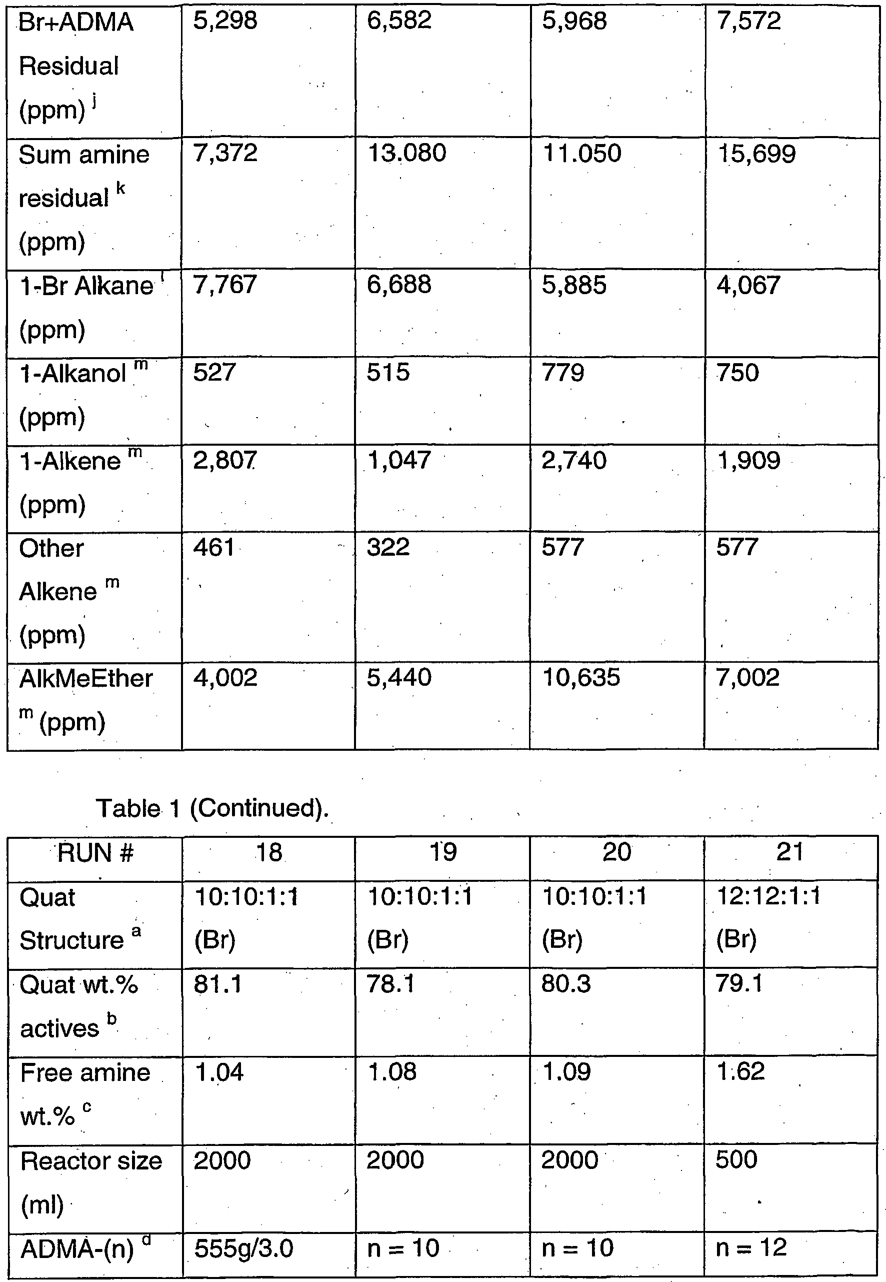

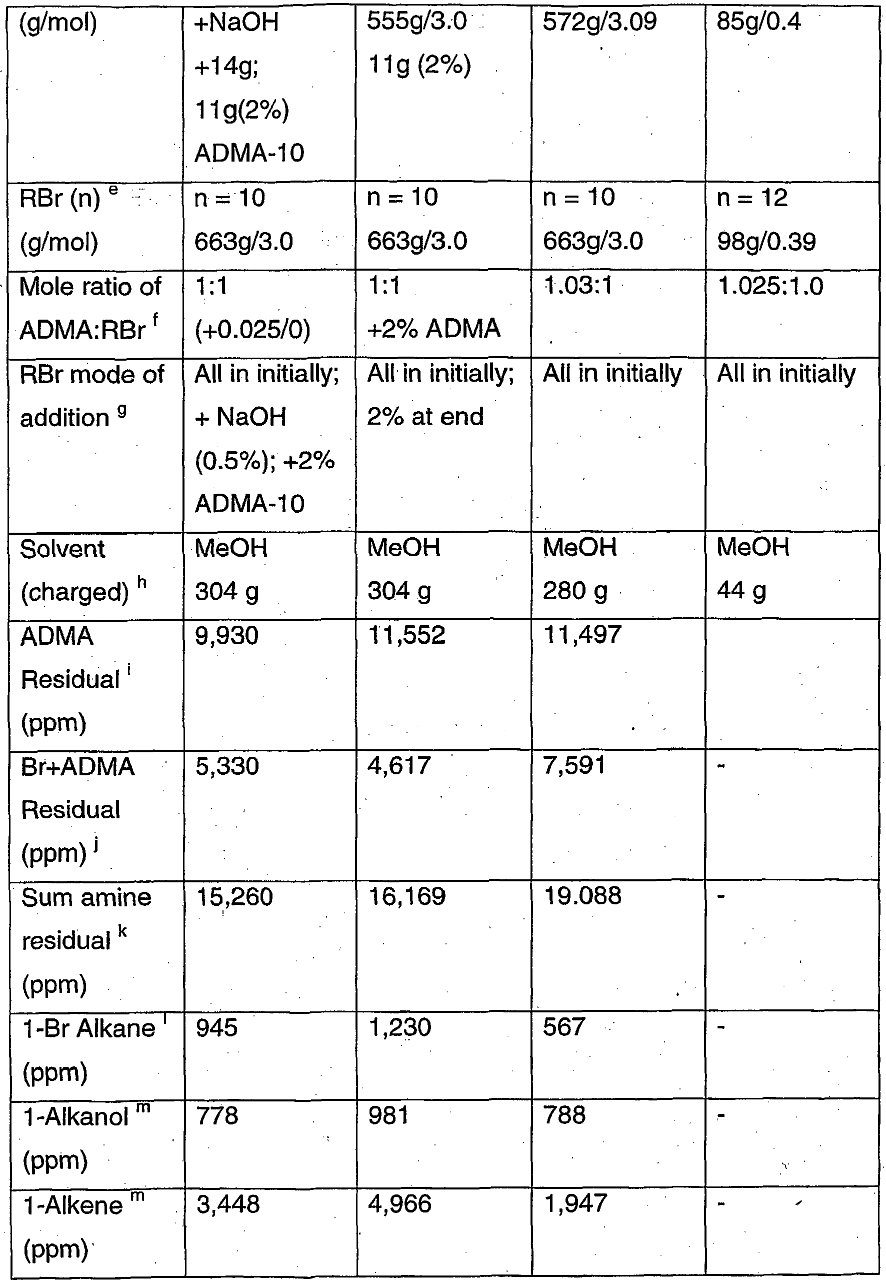

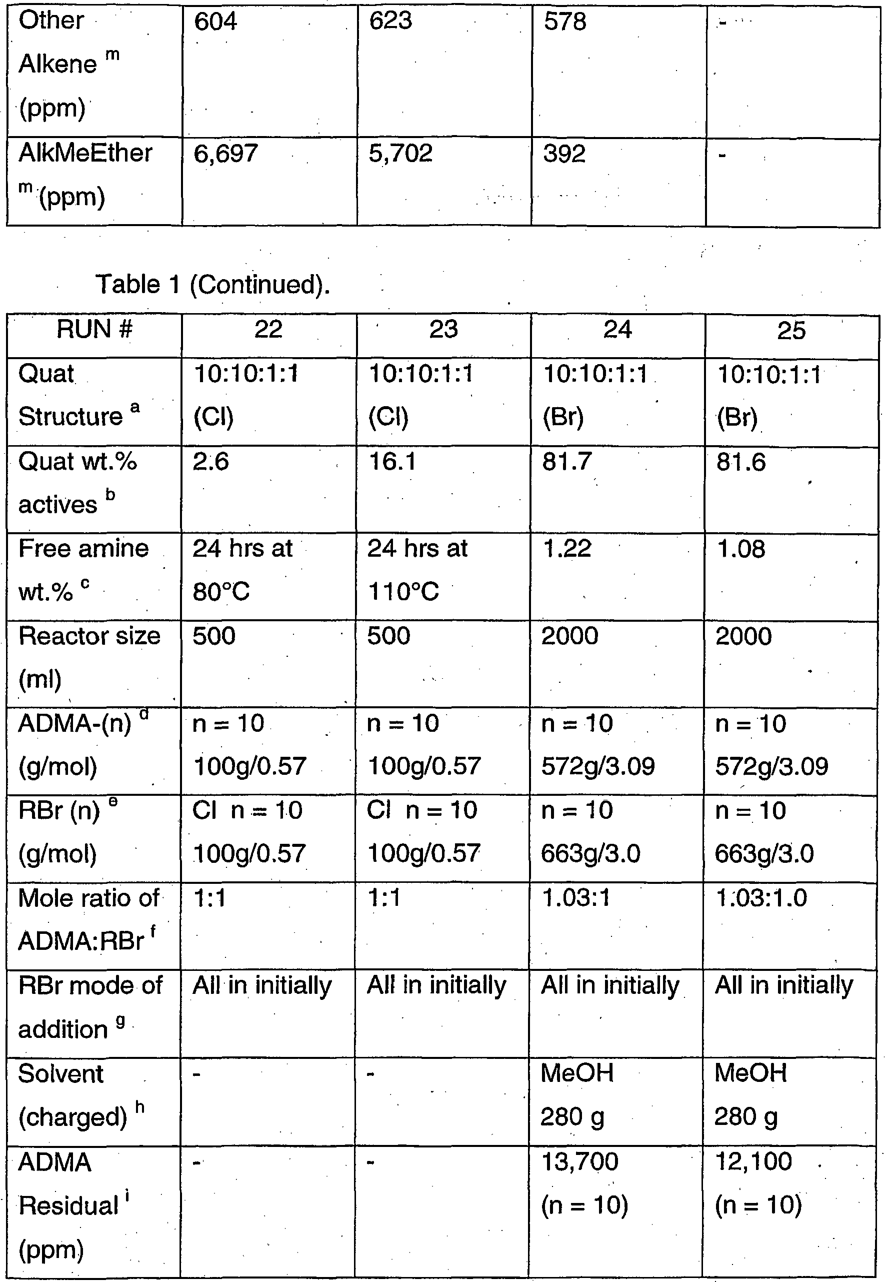

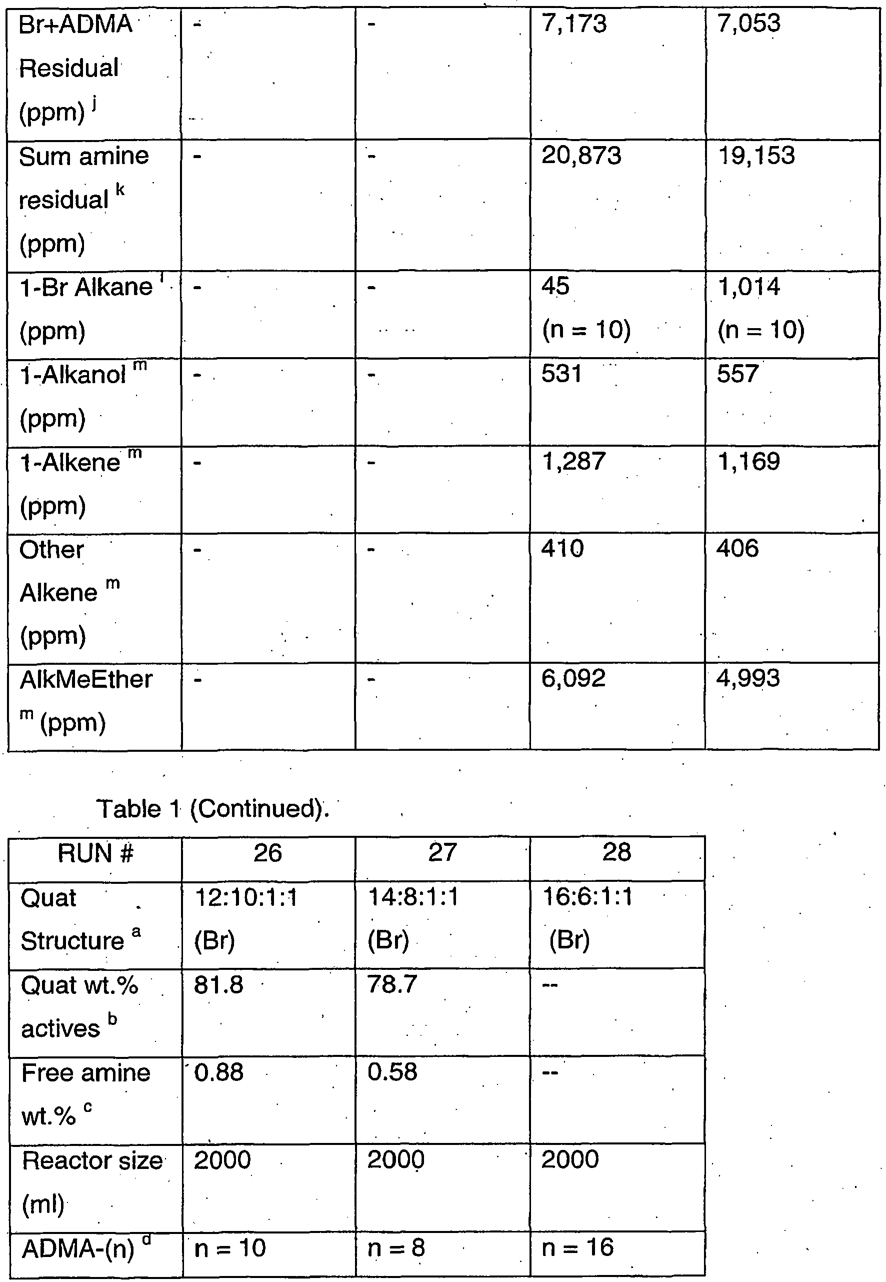

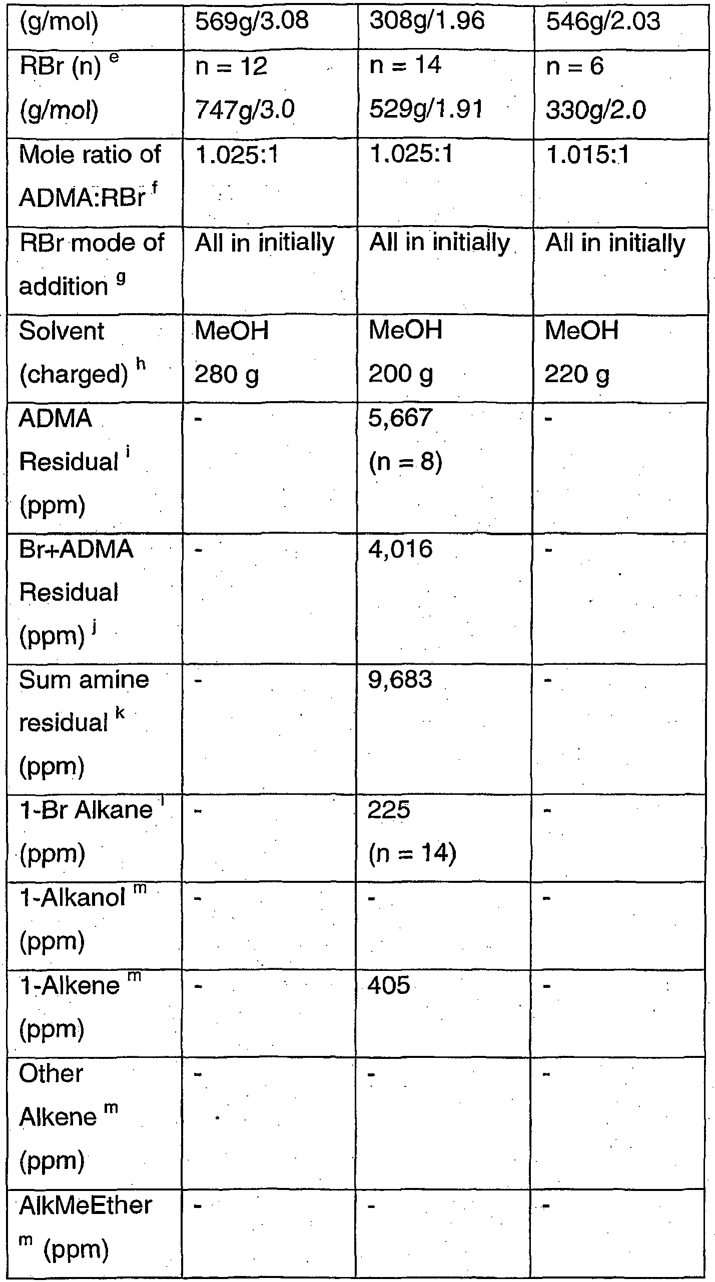

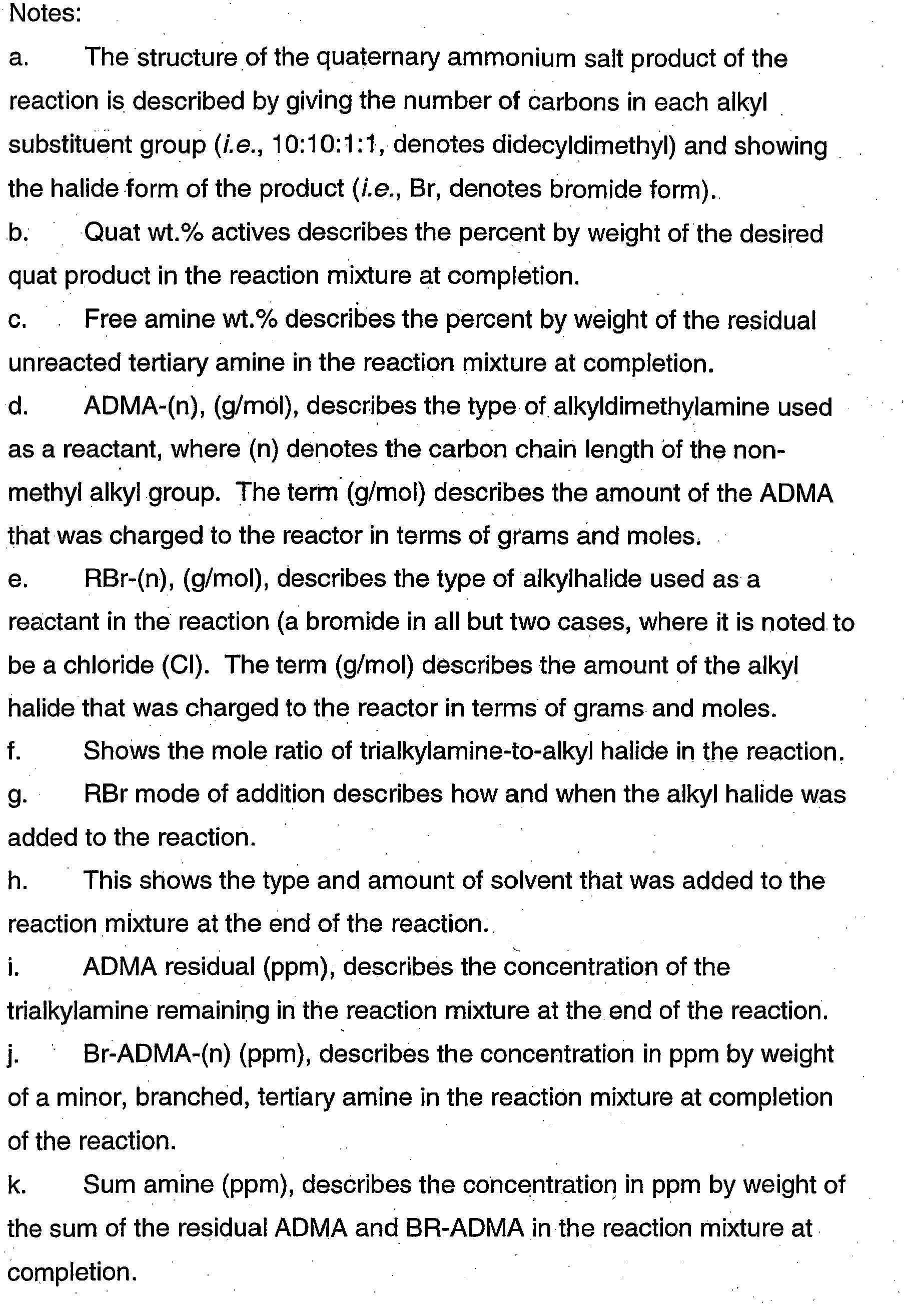

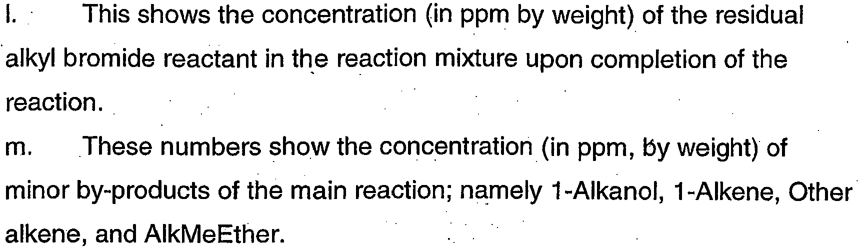

### EXAMPLES 29 - 39

These examples describe ion-exchange procedures used to exchange the halide anions, such as bromide, of a quaternary ammonium intermediate compound with other anions, such as bicarbonate.

A 28 mm x 150 mm glass chromatography column was set up with a cotton plug in the bottom. The glass column was charged with 40 grams of Amberlite A-27 Strong Base/Macroreticular Type Ion Exchange Resin (available from Rohm & Haas Company), and washed with 50 ml distilled water, pH 7. The column was charged with HCO₃⁻ ions by eluting a solution of 30 grams of NaHCO₃ in 400 ml distilled water through the column.

Five grams of quat-Br, such as that made by the process described in Example 1, was dissolved in 5 grams of methanol. This quat bromide solution was placed on the resin bed and eluted with a 2:1 mixture of methanol:water and collected in the following aliquots as depicted in Table 2.

**Table 2: One-Step Column Charging - Analysis of the Final Quat Product Eluted From an Ion-Exchange Column**

| Example/ Sample No. | Volume | pH | Visual Description |
|---|---|---|---|
| 29 | 0 ml | 8.5 | colorless |
| 30 | 25 ml | 8.0 | colorless |
| 31 | 10 ml | 7.5-8.0 | colorless |
| 32 | 10 ml | | pale yellow |
| 33 | 10 ml | 7.5-8.0 | pale yellow |
| 34 | 10 ml | | pale yellow |
| 35 | 10 ml | | pale yellow/cloudy |
| 36 | 10 ml | | cloudy |
| 37 | 10 ml | | cloudy |
| 38 | 10 ml | | very pale yellow/cloudy |
| 39 | 10 ml | 7.0 | clear |

Samples 32, 34, and 36 were selected for analysis by ion chromatography to determine the presence of residual chloride ions that were present on the resin prior to use and the presence of HCO₃⁻ ions, which is depicted in Table 3.

**Table 3: One-Step Ion Chromatography Analysis**

| **Example/ Sample No.** | **Cl⁻** | **Br⁻** | **HCO₃⁻** |
|---|---|---|---|
| 32 | 724 ppm | - | yes |
| 34 | 1011 ppm | - | yes |
| 36 | 212 ppm | - | yes |

### EXAMPLES 37 - 45

These examples describe a two-step regeneration protocol for a, previously used ion-exchange column, such as the column resulting from the procedure described in Examples 29 - 39, to remove the bromide ions present on the resin that were extracted from the intermediate quat bromide, and the subsequent use of the regenerated column for ion exchange.

The previously used resin, such as that described in Examples 29 - 39, was washed with 200 ml distilled water to purge the methanol. The column was then charged with OH⁻ ions by eluting 250 ml of 10% NaOH in distilled water through the ion exchange bed and then washed with distilled water. The resin was then charged with HCO₃⁻ ions by eluting a solution of 17.47 grams of NaHCO₃ dissolved in 300 ml of distilled water through the ion exchange bed. The column was then washed with 80 ml of distilled water, followed by washing with 95% methanol/5% water.

Then 14.52 grams of the quat-Br solution prepared in Example 1 was dissolved in 5.82 grams methanol, and introduced to the charged column. Elution was carried out with a mixture of 95%methanol/5% water, and collected in the following 15 ml aliquots detailed in Table 4.

**Table 4: One-Step Column Charging - Analysis of the Final Quat Product Eluted From a Regenerated Ion-Exchange Column**

| **Example/ Sample No.** | **Visual Description** |
|---|---|
| 37 | Discard |
| 38 | Colorless |
| 39 | Colorless |
| 40 | light yellow/cloudy |
| 41 | yellow/clear |
| 42 | yellow/clear |
| 43 | light yellow |
| 44 | very slight yellow |
| 45 | Colorless |

Samples 41 and 42 were chosen for analysis by ion chromatography to determine the presence of residual bromide ions and the presence of HCO₃⁻ ions, which is depicted in Table 5.

**Table 5: Two-step ion chromatography analysis**

| **Example/ Sample No.** | **Br⁻** | **HCO₃⁻** |
|---|---|---|
| 41 | 669 ppm | 2.53% |
| 42 | 1213 ppm | 2.65% |

### EXAMPLES 46 - 48.

These examples illustrate exchange of the bromide anion of a quaternary tetraalkylammonium bromide salt with a second anion in a 2-inch diameter column, and regeneration of the column.

### Column Preparation

Two columns, each 2" X 48", were loaded with 1500g of Dowex Marathon A2 resin which represents approximately 2.69 exchange equivalents/bed. This resin as supplied by Dow Chemical Co., Midland, MI, is in the chloride form. Each was flushed with several liters of water and then put into the desired hydroxide or bicarbonate form as follows.

### Column Degeneration

A typical regeneration to the hydroxide form involves running enough water through the column to remove any remaining organics from the previous run, typically several liters of water. This is followed by six or more liters of 8 wt% caustic, and then additional water until the effluent is neutral. Since countercurrent regeneration was difficult in the laboratory as equipped, the columns were inverted resulting in what is essentially countercurrent regeneration. After inversion, what was formerly the top of the column which had seen the most caustic and should be the most halide free, became the bottom of the column. The column was ready for use after inversion.

In some cases, recycle caustic was used in the regeneration. In that case, the later fractions of caustic used in regeneration contain little bromide and thus were used for the first caustic fractions of the next column regeneration. This was always followed by several fractions of fresh caustic.

When the bicarbonate form was desired, a two-step regeneration was employed. The column was first put into the hydroxide form as above, and then two equivalents of sodium bicarbonate in water (approximately 8 wt%) were passed through the column. Then additional water was passed until the effluent was neutral. The column was then inverted and was ready for use.

### Topical Anion Exchange Procedure

After the column is regenerated and inverted, the void volume water (approximately 50% of the column volume) has to be replaced with the solvent to be used in the exchange. Approximately one liter, more or less, of the desired solvent is passed through the column. The feed is then added and the flow rate adjusted to approximately 20 ml/min. Aliquots, generally 500 ml each at this scale, are collected and analyzed and/or combined. After all of the feed is on the column, an additional one to two liters of solvent is passed through the column to completely flush all of the quat from the column. The column is then ready for a water flush and then inversion and regeneration. All bromide values reported in Part 2 were determined by classical silver nitrate titration.

### Example 46: Conversion of Quat Bromide to Quat Bicarbonate

One column was put into the bicarbonate form and used for this experiment.

The feed for this run was 1365g of 80% didecyldimethylammonium bromide in methanol, 2457g additional methanol, and 1638g water. This resulted in a quat bromide:methanol:water ratio of 20:50:30. Initially 700 ml of methanol/water (ratio 62.5:37.5 by volume) was passed through the column to displace the water and condition the column to the correct methanol/water ratio. The feed mixture was put onto the column and was followed by a liter of methanol/water. Aliquots were collected and analyzed as shown in Table 6.

**Table 6: Residual bromide in effluent of ion exchange column as a function of volume of feed to the column.**

| Fraction | Elapsed time (min) | Volume (ml) | Flow rate (ml/min) | Bromide (ppm) | Comments |
|---|---|---|---|---|---|
| 0 | 22 | 1050 | - | | |
| 1 | 26 | 500 | 22.73 | | |
| 2 | 26 | 500 | 19.23 | | |
| 3 | 26 | 500 | 19.23 | | |
| 4 | 26 | 500 | 19.23 | 151 | Frac. 3 & 4 |
| 5 | 26 | 500 | 19.23 | | |
| 6 | 27 | 500 | 18.52 | | |
| 7 | 27 | 500 | 18.52 | | |
| 8 | 26 | 500 | 19.23 | 135 | Frac. 7 & 8 |
| 9 | 26 | 510 | 19.62 | | |
| 10 | | 550 | -- | | |
| 11 | | 480 | -- | | |
| 12 | 30 | 500 | 16.67 | 105 | Frac. 11 & 12 |
| 13 | 15 | 250 | 16.67 | | |
| 14 | 10 | 250 | 25 | 76 | Frac. 13 & 14 |

| | | | | | |
|---|---|---|---|---|---|
| Notes: Feed was 20% quat bromide, 50% methanol, 30% water. Example 47: Conversion of quat bromide to quat hydroxide with polish | | | | | |

Two columns were regenerated into the hydroxide form by the standard procedure outlined above.

The feed for this run was 1024g of 80% didecyldimethylammonium bromide in methanol, 1843g additional methanol, and 1228g water. This resulted in a quat bromide:methanol:water ratio of 20:50:30. Initially 700 ml of methanol/water (ratio 62.5:37.5 by volume) was passed through the column to displace the water and condition the column to the correct methanol/water ratio. The feed mixture was put onto the column and was followed by methanol/water. Aliquots were collected and analyzed as shown in the Tables 7 and 8, which follow.

**Table 7: Loading rate for first of two ion exchange columns.**

| Fraction | Elapsed time (min) | Volume (ml) | Flow rate (ml/min) | Bromide (ppm) | Comments |
|---|---|---|---|---|---|
| 0 | 75 | 970 | 12.93 | | |
| 1 | 44 | 500 | 11.36 | | |
| 2 | 35 | 500 | 14.29 | | |
| 3 | 35 | 500 | 14.29 | | |
| 4 | 35 | 500 | 14.29 | | |
| 5 | 34 | 500 | 14.71 | | |
| 6 | 40 | 500 | 12.50 | | |
| 7 | 36 | 500 | 13.89 | | |
| 8 | 42 | 505 | 12.02 | | |
| 9 | 39 | 500 | 12.82 | | |
| 10 | 39 | 500 | 12.82 | | |
| 11 | 34 | 340 | 10 | | |
| 12 | 30 | 500 | 16.67 | 105 | |

| | | | | | |
|---|---|---|---|---|---|
| Notes: Feed was 20% quat bromide, 50% methanol, 30% water. 75% column equivalent charge. | | | | | |

The effluent from the first column was fed to a second column, as a "polish" column, and the output from that column analyzed for residual bromide.

**Table 8: Residual bromide in effluent of second ion exchange column as a function of volume of feed to the column.**

| Fraction | Elapsed time (min) | Volume (ml) | Flow rate (ml/min) | Bromide (ppm) | Comments |
|---|---|---|---|---|---|
| 0 | 45 | 1020 | 22.67 | | |
| 1 | 35 | 500 | 14.29 | | |
| 2 | 30 | 500 | 16.67 | | |
| 3 | 30 | 500 | 16.67 | | |
| 4 | 29 | 500 | 17.24 | 164 | |
| 5 | 27 | 500 | 18.52 | | |
| 6 | 28 | 500 | 17.86 | 160 | |
| 7 | 31 | 560 | 18.06 | | |
| 8 | 26 | 440 | 16.92 | 158 | |
| 9 | 30 | 560 | 18.67 | | |
| 10 | 28 | 620 | 22.14 | 188 | |

| | | | | | |
|---|---|---|---|---|---|
| Notes: Feed was 20% quat bromide, 50% methanol, 30% water. Bromide content in combined fractions 1 -11 was 169 ppm Br. | | | | | |

### Example 48: Conversion of quat bromide to quat hydroxide at higher concentration

The feed for this run was 1279.7g of 80% didecyldimethylammonium bromide in methanol, 1843g additional methanol, and 1228g water. This resulted in a quat bromide:methanol:water ratio of 23.5:48.25:28.25. The feed quat bromide had been prepared with 1% excess amine in an effort to minimize unreacted decylbromide. The bromide concentration in the effluent from the ion exchange column is shown in Table 9.

**Table 9: Residual bromide in effluent of ion exchange column as a function of volume of feed to the column.**

| Fraction | Elapsed time (min) | Volume (ml) | Flow rate (ml/min) | Bromide (ppm) | Comments |
|---|---|---|---|---|---|
| 0 | 66 | 1030 | 15.61 | | |
| 1 | 43 | 530 | 12.33 | 223 | |
| 2 | 40 | 500 | 12.50 | 492 | |
| 3 | 40 | 560 | 14.0 | 473 | |
| 4 | 34 | 450 | 13.24 | 246 | |
| 5 | 35 | 510 | 14.57 | 267 | |
| 6 | 36 | 570 | 15.83 | 276 | |
| 7 | 32 | 500 | 15.63 | 308 | |
| 8 | 35 | 500 | 14.29 | 327 | |
| 9 | 35 | 500 | 14.29 | 7003 | |
| 10 | 30 | 490 | 16.33 | 14625 | |
| 11 | 14 | 220 | 15.71 | 3968 | Cloudy |

| | | | | | |
|---|---|---|---|---|---|
| Notes: Feed was 23.5% quat bromide, 48% methanol, 28.25% water. | | | | | |

This run clearly shows that low bromide can be obtained in a single pass through the column. Also, it clearly shows bromide breakthrough toward the end of the run. The reduced amount of bromide in fraction 11 is the result of dilution with additional methanol/water used to flush any remaining quat from the column at the end of the run.

## Claims

1. A method of producing a quaternary tetraalkylammonium salt of a second anion, the method comprising:
- reacting a trialkylamine with an alkyl bromide to form a quaternary tetraalkylammonium bromide salt;
- converting the quaternary tetraalkylammonium bromide salt to a quaternary tetraalkylammonium hydroxide salt by
- contacting an ion exchange resin in hydroxide form with a solution comprising
- the quaternary tetraalkylammonium bromide salt in an amount of above at least 10% by weight and
- a solvent that includes water and a polar organic co-solvent and has a ratio of co-solvent to water of 10:90 to 99:1, and
forming the quaternary tetraalkylammonium hydroxide salt in solution and the ion exchange resin in bromide form,
whereby contacting the ion exchange resin in hydroxide form with the solution comprising the quaternary tetraalkylammonium bromide salt comprises flowing the solution containing the tetraalkylammonium bromide salt into a vessel containing a bed of the ion exchange resin in hydroxide form so that the solution passes through the resin bed causing the bromide anions to exchange with the hydroxide anions to form quaternary tetraalkylammonium hydroxide salt and ion exchange resin in bromide form; and
- converting the quaternary tetraalkylammonium hydroxide salt to the quaternary tetraalkylammonium salt of the second anion.

2. The method according to claim 1, further comprising recovering the quaternary tetraalkylammonium salt of the second anion.

3. The method according to claim 1, wherein the trialkylamine is an alkyldimethylamine.

4. The method according to claim 1, wherein the trialkylamine is a (C₁-C₂₀) alkyldimethylamine.

5. The method according to claim 1, wherein the trialkylamine is decyldimethylamine.

6. The method according to claim 1, wherein the alkyl bromide is a (C₁-C₂₀) alkyl bromide.

7. The method according to claim 1, wherein the alkyl bromide is decyl bromide.

8. The method according to claim 1, wherein the trialkylamine is decyldimethylamine and the alkyl bromide is decyl bromide and the tetraalkylammonium bromide salt is didecyldimethylammonium bromide.

9. The method according to claim 1, wherein the trialkylamine is reacted with the decyl bromide in the presence of a molar excess of the trialkylamine.

10. The method according to claim 1, wherein the trialkylamine and the alkyl bromide are initially present respectively in a mole ratio that is greater than 1.0.

11. The method according to claim 10, wherein the mole ratio is between 1.0001:1 and 2:1.

12. The method according to claim 10, wherein the mole ratio is between 1.01:1 and 1.02:1.

13. The method according to claim 1, wherein the solution containing the tetraalkylammonium hydroxide salt comprises less than 3000 ppm of bromide.

14. The method according to claim 1, wherein the solution containing the tetraalkylammonium hydroxide salt comprises less than 1000 ppm of bromide.

15. The method according to claim 1, wherein the solution containing the tetraalkylammonium hydroxide salt comprises less than 300 ppm of bromide.

16. The method according to claim 1, wherein the solution containing the tetraalkylammonium hydroxide salt comprises less than 300 ppm of bromide reported by weight as chloride.

17. The method according to claim 1, further comprising regenerating the ion exchange resin in bromide form by contacting it with sodium hydroxide and forming sodium bromide and the ion exchange resin in hydroxide form.

18. The method according to claim 1, wherein the solution comprises the tetraalkylammonium bromide salt in a concentration of that is above at least 10% by weight.

19. The method according to claim 1, wherein the solution comprises the tetraalkylammonium bromide salt in a concentration of that is above at least 20% by weight.

20. The method according to claim 1, wherein the solvent is one in which:
(i) the tetraalkylammonium bromide salt is soluble in an amount of at least 1 % by weight at 25°C,
(ii) the tetraalkylammonium hydroxide salt is soluble in an amount of at least 1 % by weight at 25°C,
(iii)the bromide is available for exchange.

21. The method according to claim 20, wherein the solvent is one in which the bromide is available for exchange is one in which:
- the tetraalkylammonium bromide salt and the ion exchange resin in hydroxide form are ionized, at least to some degree; and
- the tetraalkylammonium bromide salt does not form molecular assemblies that shield the bromide from contact with the ion exchange resin.

22. The method according to claim 1, wherein the solvent comprises a mixture of alcohol and water.

23. The method according to claim 1, wherein the solvent comprises a mixture of a C₁-C₆ alcohol and water in a ratio of from 10:90 to 99:1 by weight.

24. The method according to claim 1, wherein the solvent comprises a mixture of a C₁-C₄ alcohol and water in a ratio of from 50:50 to 99:1 by weight.

25. The method according to claim 1, wherein the solvent comprises a mixture of methanol and water in a ratio of from 70:30 to 99:1 by weight.

26. The method according to claim 1, wherein the step of converting the quaternary tetraalkylammonium hydroxide salt to the quaternary tetraalkylammonium salt of the second anion comprises contacting the solution of the tetraalkylammonium hydroxide salt with an ion exchange resin in the form of the second anion and forming a tetraalkylammonium salt of the second anion in solution and the ion exchange resin in hydroxide form.

27. The method according to claim 26, wherein at least 90 mol% of the tetraalkylammonium hydroxide salt is converted to the tetraalkylammonium salt of the second anion.

28. The method according to claim 26, wherein at least 99 mol% of the tetraalkylammonium hydroxide salt is converted to the tetraalkylammonium salt of the second anion.

29. The method according to claim 26, wherein at least 99.9 mol% of the tetraalkylammonium hydroxide salt is converted to the tetraalkylammonium salt of the second anion.

30. The method according to claim 26, wherein the second anion comprises at least one anion except for bromide that is selected from the group consisting of halide, hydroxide, borate, formate, carboxylates, carbonate, bicarbonate, sulfate, bisulfate, sulfite, sulfonate, phosphate, phosphonate, nitrate, chlorate, acetate, and mixtures thereof.

31. The method according to claim 26, wherein the second anion is carbonate or a mixture of carbonate and bicarbonate.

32. The method according to claim 1, wherein the step of converting the quaternary tetraalkylammonium hydroxide salt to the quaternary tetraalkylammonium salt of the second anion comprises contacting the tetraalkylammonium hydroxide salt with an acid of the second anion and converting the tetraalkylammonium hydroxide salt to a tetraalkylammonium salt of the second anion.

33. The method according to claim 32, wherein the acid of the second anion comprises an acid having as an anion at least one anion except for bromide that is selected from the group consisting of halide, borate, formate, carboxylates, carbonate, bicarbonate, sulfate, bisulfate, sulfite, sulfonate, phosphate, phosphonate, nitrate, chlorate, acetate, and mixtures thereof.

34. The method according to claim 33, wherein the acid of the second anion is carbonic acid that is formed by contacting carbon dioxide with an aqueous solution to form carbonate or a mixture of carbonate and bicarbonate anions.

35. The method according to claim 32, wherein the step of contacting the tetraalkylammonium hydroxide salt with an acid of the second anion and converting at least a portion of the tetraalkylammonium hydroxide salt to a tetraalkylammonium salt of the second anion is performed in a reactive distillation column.

36. The method according to claim 35, wherein the reactive distillation column is fed the solution comprising the tetraalkylammonium hydroxide salt and is also fed carbon dioxide at a point where the carbon dioxide will flow up the column thereby forming carbonic acid which reacts with the tetraalkylammonium hydroxide salt and forms a tetraalkylammonium carbonate and/or bicarbonate salt which exits the bottom of the column in an aqueous solution, and wherein the co-solvent is stripped from the feed solution and exits the top of the column.

37. The method according to claim 34, wherein the tetraalkylammonium salt of a second anion comprises didecyldimethylammonium carbonate and didecyldimethylammonium bicarbonate.

38. The method according to claim 37, wherein the tetraalkylammonium salt of a second anion comprises didecyldimethylammonium carbonate and didecyldimethylammonium bicarbonate in a molar ratio of 10:90.

39. The method according to claim 34, wherein the tetraalkylammonium hydroxide salt is didecyldimethyl-ammonium hydroxide salt and the aqueous solution containing the didecyldimethylammonium hydroxide salt is contacted with a sufficient amount of carbon dioxide to produce a mixture of 10% didecyldimethylammonium carbonate and 90% didecyldimethylammonium bicarbonate on a molar basis.

## Patentansprüche

1. Verfahren zur Herstellung von quartärem Tetraalkyl-ammoniumsalz eines zweiten Anions, wobei das Verfahren umfasst:
- Umsetzen eines Trialkylamins mit einem Alkylbromid, um ein quartäres Tetraalkylammoniumbromidsalz zu bilden,
- Umwandeln des quartären Tetraalkylammoniumbromidsalzes in ein quartäres Tetraalkylammoniumhydroxidsalz durch
- In-Kontakt-Bringen eines Ionenaustauschharzes in Hydroxidform mit einer Lösung, die
- das quartäre Tetraalkylammoniumbromidsalz in einer Menge oberhalb von mindestens 10 Gew.-% und
- Lösungsmittel umfasst, das Wasser und ein polares organisches Co-Lösungsmittel enthält und ein Verhältnis von Co-Lösungsmittel zu Wasser von 10:90 bis 99:1 aufweist, und
Bilden des quartären Tetraalkylammoniumhydroxidsalzes in Lösung und des Ionenaustauschharzes in Bromidform,
wobei das In-Kontakt-Bringen des Ionenaustauschharzes in Hydroxidform mit der Lösung, die das quartäre Tetraalkylammoniumbromidsalz umfasst, das Fließen der Lösung, die das Tetraalkylammoniumbromidsalz enthält, in ein Gefäß umfasst, das ein Bett aus dem Ionenaustauschharz in Hydroxidform enthält, so dass die Lösung durch das Harzbett läuft, was dazu führt, dass die Bromidanionen durch die Hydroxidanionen ausgetauscht werden, um quartäres Tetraalkylammoniumhydroxidsalz und Ionenaustauschharz in Bromidform zu bilden, und
- Umwandeln des quartären Tetraalkylammoniumhydroxidsalzes in das quartäre Tetraalkylammoniumsalz des zweiten Anions.

2. Verfahren nach Anspruch 1, bei dem ferner das quartäre Tetraalkylammoniumsalz des zweiten Anions gewonnen wird.

3. Verfahren nach Anspruch 1, bei dem das Trialkylamin ein Alkyldimethylamin ist.

4. Verfahren nach Anspruch 1, bei dem das Trialkylamin ein (C₁-C₂₀) -Alkyldimethylamin ist.

5. Verfahren nach Anspruch 1, bei dem das Trialkylamin Decyldimethylamin ist.

6. Verfahren nach Anspruch 1, bei dem das Alkylbromid ein (C₁-C₂₀) -Alkylbromid ist.

7. Verfahren nach Anspruch 1, bei dem das Alkylbromid Decylbromid ist.

8. Verfahren nach Anspruch 1, bei dem das Trialkylamin Decyldimethylamin ist und das Alkylbromid Decylbromid ist und das Tetraalkylammoniumbromidsalz Didecyldimethylammoniumbromid ist.

9. Verfahren nach Anspruch 1, bei dem das Trialkylamin mit dem Decylbromid in Gegenwart eines molaren Überschusses des Trialkylamins umgesetzt wird.

10. Verfahren nach Anspruch 1, bei dem das Trialkylamin und das Alkylbromid anfangs jeweils in einem Molverhältnis vorliegen, das größer als 1,0 ist.

11. Verfahren nach Anspruch 10, bei dem das Molverhältnis 1,0001 : 1 bis 2:1 beträgt.

12. Verfahren nach Anspruch 10, bei dem das Molverhältnis 1,01:1 bis 1,02 : 1 beträgt.

13. Verfahren nach Anspruch 1, bei dem die Lösung, die das Tetraalkylammoniumhydroxidsalz enthält, weniger als 3000 ppm Bromid umfasst.

14. Verfahren nach Anspruch 1, bei dem die Lösung, die das Tetraalkylammoniumhydroxidsalz enthält, weniger als 1000 ppm Bromid umfasst.

15. Verfahren nach Anspruch 1, bei dem die Lösung, die das Tetraalkylammoniumhydroxidsalz enthält, weniger als 300 ppm Bromid umfasst.

16. Verfahren nach Anspruch 1, bei dem die Lösung, die das Tetraalkylammoniumhydroxidsalz enthält, weniger als 300 ppm Bromid umfasst, angegeben in Gewicht als Chlorid.

17. Verfahren nach Anspruch 1, bei dem ferner das Ionenaustauschharz in Bromidform regeneriert wird, indem es mit Natriumhydroxid in Kontakt gebracht wird und Natriumbromid und das Ionenaustauschharz in Hydroxidform gebildet werden.

18. Verfahren nach Anspruch 1, bei dem die Lösung das Tetraalkylammoniumbromidsalz in einer Konzentration umfasst, die oberhalb von mindestens 10 Gew.-% liegt.

19. Verfahren nach Anspruch 1, bei dem die Lösung das Tetraalkylammoniumbromidsalz in einer Konzentration umfasst, die oberhalb von mindestens 20 Gew.-% liegt.

20. Verfahren nach Anspruch 1, bei dem das Lösungsmittel eines ist, in dem
(i) das Tetraalkylammoniumbromidsalz in einer Menge von mindestens 1 Gew.-% bei 25°C löslich ist,
(ii) das Tetraalkylammoniumhydroxidsalz in einer Menge von mindestens 1 Gew.-% bei 25°C löslich ist,
(iii)das Bromid für den Austausch zur Verfügung steht.

21. Verfahren nach Anspruch 20, bei dem das Lösungsmittel eines ist, indem das Bromid für den Austausch zur Verfügung steht, eines ist, in dem
- das Tetraalkylammoniumbromidsalz und das Ionenaustauschharz in Hydroxidform ionisiert sind, zumindest in gewissem Ausmass, und
- das Tetraalkylammoniumbromidsalz keine molekularen Ansammlungen bildet, die das Bromid von dem Kontakt mit dem Ionenaustauschharz abschirmen.

22. Verfahren nach Anspruch 1, bei dem das Lösungsmittel eine Mischung aus Alkohol und Wasser umfasst.

23. Verfahren nach Anspruch 1, bei dem das Lösungsmittel eine Mischung aus einem C₁-C₆-Alkohol und Wasser in einem Verhältnis von 10:90 bis 99:1 umfasst, bezogen auf das Gewicht.

24. Verfahren nach Anspruch 1, bei dem das Lösungsmittel eine Mischung aus einem C₁-C₄-Alkohol und Wasser in einem Verhältnis von 50:50 bis 99:1 umfasst, bezogen auf das Gewicht.

25. Verfahren nach Anspruch 1, bei dem das Lösungsmittel eine Mischung aus Methanol und Wasser in einem Verhältnis von 70:30 bis 99:1 umfasst, bezogen auf das Gewicht.

26. Verfahren nach Anspruch 1, bei dem der Schritt der Umwandlung des quartären Tetraalkylammoniumhydroxidsalzes in das quartäre Tetraalkylammoniumsalz des zweiten Anions, das In-Kontakt-Bringen der Lösung des Tetraalkylammoniumhydroxidsalzes mit einem Ionenaustauschharz in Form des zweiten Anions und das Bilden eines Tetraalkylammoniumsalzes des zweiten Anions in Lösung und des Ionenaustauschharzes in Hydroxidform umfasst.

27. Verfahren nach Anspruch 26, bei dem mindestens 90 Mol.-% des Tetraalkylammoniumhydroxidsalzes in das Tetraalkylammoniumsalz des zweiten Anions umgewandelt werden.

28. Verfahren nach Anspruch 26, bei dem mindestens 99 Mol.-% des Tetraalkylammoniumhydroxidsalzes in das Tetraalkylammoniumsalz des zweiten Anions umgewandelt werden.

29. Verfahren nach Anspruch 26, bei dem mindestens 99,9 Mol.-% des Tetraalkylammoniumhydroxidsalzes in das Tetraalkylammoniumsalz des zweiten Anions umgewandelt werden.

30. Verfahren nach Anspruch 26, bei dem das zweite Anion mindestens eine Anion, ausgenommen Bromid, umfasst, das ausgewählt ist aus der Gruppe bestehend aus Halogenid, Hydroxid, Borat, Formiat, Carboxylaten, Carbonat, Bicarbonat, Sulfat, Bisulfat, Sulfit, Sulfonat, Phosphat, Phosphonat, Nitrat, Chlorat, Acetat und Mischungen derselben.

31. Verfahren nach Anspruch 26, bei dem das zweite Anion Carbonat und eine Mischung von Carbonat und Bicarbonat ist.

32. Verfahren nach Anspruch 1, bei dem der Schritt des Umwandelns des quartären Tetraalkylammoniumhydroxidsalzes in das quartäre Tetraalkylammoniumsalz des zweiten Anions und das In-Kontakt-Bringen des Tetraalkylammoniumhydroxidsalzes mit einer Säure des zweiten Anions und das Umwandeln des Tetraalkylammoniumhydroxidsalzes in ein Tetraalkylammoniumsalz des zweiten Anions umfasst.

33. Verfahren nach Anspruch 32, bei dem die Säure des zweiten Anions eine Säure umfasst, die als Anion mindestens ein Anion, ausgenommen Bromid, aufweist, das ausgewählt ist aus der Gruppe bestehend aus Halogenid, Borat, Formiat, Carboxylaten, Carbonat, Bicarbonat, Sulfat, Bisulfat, Sulfit, Sulfonat, Phosphat, Phosphonat, Nitrat, Chlorat, Acetat und Mischungen derselben.

34. Verfahren nach Anspruch 33, bei dem die Säure des zweiten Anions Carbonsäure ist, die durch In-Kontakt-Bringen von Kohlendioxid mit einer wässrigen Lösung gebildet ist, um Carbonat oder eine Mischung von Carbonat- und Bicarbonatanionen zu bilden.

35. Verfahren nach Anspruch 32, bei dem der Schritt des In-Kontakt-Bringens des Tetraalkylammoniumhydroxidsalzes mit einer Säure des zweiten Anions und des Umwandelns mindestens eines Teil des Tetraalkylammoniumhydroxidsalzes in ein Tetraalkylammoniumsalz des zweiten Anions in einer reaktiven Destillationssäule durchgeführt wird.

36. Verfahren nach Anspruch 35, bei dem die reaktive Destillationssäule mit der Lösung beschickt wird, die das Tetraalkylammoniumhydroxidsalz umfasst, und außerdem mit Kohlendioxid an einem Punkt beschickt wird, an dem das Kohlendioxid in der Säule aufwärts strömt, wodurch Carbonsäure gebildet wird, die mit dem Tetraalkylammoniumhydroxidsalz reagiert und ein Tetraalkylammoniumcarbonat- und/oder -bicarbonatsalz bildet, das aus dem Boden der Säule in einer wässrigen Lösung austritt, und bei dem das Co-Lösungsmittel von der Einsatzmateriallösung entfernt wird und den Kopf der Säule verlässt.

37. Verfahren nach Anspruch 34, bei dem das Tetraalkylammoniumsalz eines zweiten Anions Didecyldimethylammoniumcarbonat und Didecyldimethylammoniumbicarbonat umfasst.

38. Verfahren nach Anspruch 37, bei dem das Tetraalkylammoniumsalz eines zweiten Anions Didecyldimethylammoniumcarbonat und Didecyldimethylammoniumdicarbonat in einem Molverhältnis von 10:90 umfasst.

39. Verfahren nach Anspruch 34, bei dem das Tetraalkylammoniumhydroxidsalz Didecyldimethylammoniumhydroxidsalz ist und die wässrige Lösung, die das Didecyldimethylammoniumhydroxidsalz enthält, mit einer ausreichenden Menge Kohlendioxid in Kontakt gebracht wird, um auf Molbasis eine Mischung aus 10% Didecyldimethylammoniumcarbonat und 90% Didecyldimethylammoniumbicarbonat zu bilden.

## Revendications

1. Procédé de production d'un sel de tétraalkylammonium quaternaire d'un second anion, le procédé comprenant :
- la réaction d'une trialkylamine avec un bromure d'alkyle pour former un sel de bromure de tétraalkylammonium quaternaire ;
- la conversion du sel de bromure de tétra-alkylammonium quaternaire en sel d'hydroxyde de tétra-alkylammonium quaternaire par
- le contact d'une résine échangeuse d'ions sous forme hydroxyde avec une solution comprenant
- le sel de bromure de tétraalkylammonium quaternaire en une quantité supérieure à au moins 10% en poids et
- un solvant qui contient de l'eau et un cosolvant organique polaire et présente un rapport cosolvant sur eau de 10 : 90 à 99 : 1, et
la formation du sel d'hydroxyde de tétraalkylammonium quaternaire en solution et de la résine échangeuse d'ions sous forme de bromure,
moyennant quoi le contact de la résine échangeuse d'ions sous forme d'hydroxyde avec la solution comprenant le sel de bromure de tétraalkylammonium quaternaire comprend l'écoulement de la solution contenant le sel de bromure de tétraalkylammonium dans un récipient contenant un lit de la résine échangeuse d'ions sous forme d'hydroxyde de façon que la solution passe à travers le lit de résine afin que les anions de bromure s'échangent avec les anions d'hydroxyde pour former un sel d'hydroxyde de tétra-alkylammonium quaternaire et la résine échangeuse d'ions sous forme de bromure ; et
- la conversion du sel d'hydroxyde de tétra-alkylammonium quaternaire en sel de tétraalkylammonium quaternaire du second anion.

2. Procédé selon la revendication 1, comprenant en outre la récupération du sel de tétraalkylammonium quaternaire du second anion.

3. Procédé selon la revendication 1, dans lequel la trialkylamine est une alkyldiméthylamine.

4. Procédé selon la revendication 1, dans lequel la trialkylamine est une (alkyl en C₁ à C₂₀)-diméthylamine.

5. Procédé selon la revendication 1, dans lequel la trialkylamine est la décyldiméthylamine.

6. Procédé selon la revendication 1, dans lequel le bromure d'alkyle est un bromure d'alkyle en C₁ à C₂₀.

7. Procédé selon la revendication 1, dans lequel le bromure d'alkyle est le bromure de décyle.

8. Procédé selon la revendication 1, dans lequel la trialkylamine est la décyldiméthylamine et le bromure d'alkyle est le bromure de décyle et le sel de bromure de tétraalkylammonium est le bromure de didécyldiméthylammonium.

9. Procédé selon la revendication 1, dans lequel la trialkylamine est mise à réagir avec le bromure de décyle en présence d'un excès molaire de la trialkylamine.

10. Procédé selon la revendication 1, dans lequel la trialkylamine et le bromure d'alkyle sont initialement présents respectivement selon un rapport molaire qui est supérieur à 1,0.

11. Procédé selon la revendication 10, dans lequel le rapport molaire est situé entre 1,0001 : 1 et 2 : 1.

12. Procédé selon la revendication 10, dans lequel le rapport molaire est situé entre 1,01 : 1 et 1,02 : 1.

13. Procédé selon la revendication 1, dans lequel la solution contenant le sel d'hydroxyde de tétraalkylammonium comprend moins de 3000 ppm de brome.

14. Procédé selon la revendication 1, dans lequel la solution contenant le sel d'hydroxyde de tétraalkylammonium comprend moins de 1000 ppm de brome.

15. Procédé selon la revendication 1, dans lequel la solution contenant le sel d'hydroxyde de tétraalkylammonium comprend moins de 300 ppm de brome.

16. Procédé selon la revendication 1, dans lequel la solution contenant le sel d'hydroxyde de tétraalkylammonium comprend moins de 300 ppm de brome rapporté en poids en termes de chlorure.

17. Procédé selon la revendication 1, comprenant en outre la régénération de la résine échangeuse d'ions sous forme de bromure par son contact avec de l'hydroxyde de sodium et la formation de bromure de sodium et de la résine échangeuse d'ions sous forme d'hydroxyde.

18. Procédé selon la revendication 1, dans lequel la solution comprend le sel de bromure de tétraalkylammonium à une concentration qui est supérieure à au moins 10% en poids.

19. Procédé selon la revendication 1, dans lequel la solution comprend le sel de bromure de tétraalkylammonium à une concentration qui est supérieure à au moins 20% en poids.

20. Procédé selon la revendication 1, dans lequel le solvant est un solvant dans lequel :
(i) le sel de bromure de tétraalkylammonium est soluble en une quantité d'au moins 1% en poids à 25°C,
(ii) le sel d'hydroxyde de tétraalkylammonium est soluble en une quantité d'au moins 1% en poids à 25°C,
(iii) le bromure est disponible pour un échange.

21. Procédé selon la revendication 20, dans lequel le solvant dans lequel le bromure est disponible pour un échange est un solvant dans lequel :
- le sel de bromure de tétraalkylammonium et la résine échangeuse d'ions sous forme d'hydroxyde sont ionisés, au moins jusqu'à un certain degré ; et
- le sel de bromure de tétraalkylammonium ne forme pas d'assemblages moléculaires qui empêchent le bromure d'entrer en contact avec la résine échangeuse d'ions.

22. Procédé selon la revendication 1, dans lequel le solvant comprend un mélange d'alcool et d'eau.

23. Procédé selon la revendication 1, dans lequel le solvant comprend un mélange contenant un alcool en C₁ à C₆ et de l'eau selon un rapport allant de 10 : 90 à 99 : 1 en poids.

24. Procédé selon la revendication 1, dans lequel le solvant comprend un mélange contenant un alcool en C₁ à C₄ et de l'eau selon un rapport allant de 50 : 50 à 99 : 1 en poids.

25. Procédé selon la revendication 1, dans lequel le solvant comprend un mélange contenant du méthanol et de l'eau selon un rapport allant de 70 : 30 à 99 : 1 en poids.

26. Procédé selon la revendication 1, dans lequel l'étape de conversion du sel d'hydroxyde de tétraalkylammonium quaternaire en sel de tétraalkylammonium quaternaire du second anion comprend le contact de la solution du sel d'hydroxyde de tétraalkylammonium avec une résine échangeuse d'ions sous la forme du second anion et la formation d'un sel de tétraalkylammonium du second anion en solution et de la résine échangeuse d'ions sous forme d'hydroxyde.

27. Procédé selon la revendication 26, dans lequel au moins 90% en mole du sel d'hydroxyde de tétraalkylammonium sont convertis en sel de tétraalkylammonium du second anion.

28. Procédé selon la revendication 26, dans lequel au moins 99% en mole du sel d'hydroxyde de tétraalkylammonium sont convertis en sel de tétraalkylammonium du second anion.

29. Procédé selon la revendication 26, dans lequel au moins 99,9% en mole du sel d'hydroxyde de tétraalkylammonium sont convertis en sel de tétraalkylammonium du second anion.

30. Procédé selon la revendication 26, dans lequel le second anion comprend au moins un anion, à l'exception du bromure, qui est choisi dans le groupe constitué par les halogénures, l'hydroxyde, le borate, le formiate, les carboxylates, le carbonate, le bicarbonate, le sulfate, le bisulfate, le sulfite, le sulfonate, le phosphate, le phosphonate, le nitrate, le chlorate, l'acétate et leurs mélanges.

31. Procédé selon la revendication 26, dans lequel le second anion est le carbonate ou un mélange de carbonate et de bicarbonate.

32. Procédé selon la revendication 1, dans lequel l'étape de conversion du sel d'hydroxyde de tétraalkylammonium quaternaire en sel de tétraalkylammonium quaternaire du second anion comprend le contact du sel d'hydroxyde de tétraalkylammonium avec un acide du second anion et la conversion du sel d'hydroxyde de tétraalkylammonium en sel de tétraalkylammonium du second anion.

33. Procédé selon la revendication 32, dans lequel l'acide du second anion comprend un acide contenant comme anion au moins un anion, à l'exception du bromure, qui est choisi dans le groupe constitué par les halogénures, le borate, le formiate, les carboxylates, le carbonate, le bicarbonate, le sulfate, le bisulfate, le sulfite, le sulfonate, le phosphate, le phosphonate, le nitrate, le chlorate, l'acétate et leurs mélanges.

34. Procédé selon la revendication 33, dans lequel l'acide du second anion est l'acide carbonique qui est formé par le contact du dioxyde de carbone avec une solution aqueuse pour former un carbonate ou un mélange d'anions de carbonate et de bicarbonate.

35. Procédé selon la revendication 32, dans lequel l'étape de contact du sel d'hydroxyde de tétraalkylammonium avec un acide du second anion et de conversion d'au moins une partie du sel d'hydroxyde de tétraalkylammonium en sel de tétraalkylammonium du second anion est réalisée dans une colonne de distillation réactive.

36. Procédé selon la revendication 35, dans lequel la colonne de distillation réactive est alimentée avec la solution comprenant le sel d'hydroxyde de tétraalkylammonium et est également alimentée en dioxyde de carbone à un endroit où le dioxyde de carbone va s'écouler vers le haut de la colonne, pour ainsi former de l'acide carbonique qui réagit avec le sel d'hydroxyde de tétraalkylammonium et forme un sel de carbonate et/ou de bicarbonate de tétraalkylammonium qui sort par le bas de la colonne dans une solution aqueuse, et dans lequel le cosolvant est retiré de la solution d'alimentation et sort par le haut de la colonne.

37. Procédé selon la revendication 34, dans lequel le sel de tétraalkylammonium du second anion comprend le carbonate de didécyldiméthylammonium et le bicarbonate de didécyldiméthylammonium.

38. Procédé selon la revendication 37, dans lequel le sel de tétraalkylammonium du second anion comprend le carbonate de didécyldiméthylammonium et le bicarbonate de didécyldiméthylammonium à un rapport molaire de 10 : 90.

39. Procédé selon la revendication 34, dans lequel le sel d'hydroxyde de tétraalkylammonium est le sel d'hydroxyde de didécyldiméthylammonium et la solution aqueuse contenant le sel d'hydroxyde de didécyldiméthylammonium est mise en contact avec une quantité suffisante de dioxyde de carbone pour produire un mélange contenant 10% de carbonate de didécyldiméthylammonium et 90% de bicarbonate de didécyldiméthylammonium sur une base molaire.
